# EUROPEAN PATENT APPLICATION

(11) **EP 4 729 623 A1**
(43) Date of publication of application: **22.04.2026**
(21) Application number: 24822769.6
(22) Date of filing: 14.06.2024
(51) Int. Cl.: C12P 7/64, C12P 7/6409, A23L 33/115, A61P 1/00, A61P 5/50, A61P 21/00, A61P 29/00

(54) **SUBSTANCE AND METHOD FOR IMPROVING INTESTINAL HEALTH AND HOMEOSTASIS, AND NUTRITION AND DEVELOPMENT OF SUBJECT**

(30) Priority: 16.06.2023 CN 202310721012
(71) Applicant: Wilmar (shanghai) Biotechnology Research & Development Center Co., Ltd., Shanghai 200137 (CN); Wilmar International Limited, Singapore 138568 (SG)
(72) Inventor: CHUA, Nam Hai, Singapore 138568 (SG); CONG, Fang, Shanghai 200137 (CN); LI, Mengmeng, Shanghai 200137 (CN); CAO, Bo, Shanghai 200137 (CN); DUAN, Yehui, Changsha, Hunan 410125 (CN); YIN, Yulong, Changsha, Hunan 410125 (CN); WANG, Junjun, Beijing 100193 (CN); LI, Yi, Beijing 100193 (CN); ZHANG, Hongfang, Singapore 138568 (SG)
(74) Representative: Aera A/S
(86) International application number: PCT/CN2024/099133
(87) International publication number: WO 2024/255821

(57) **Abstract**

Provided herein are substances and methods for improving the health and homeostasis of the intestinal tract, and the nutrition and development of a subject. The substances are odd-carbon fatty acids. Specifically, provided herein are uses of odd-carbon fatty acids in the preparation of formulations or medicaments for, among other things, increasing the protein expression abundance of a tight junction-associated protein in a subject, reducing the content of an adrenocorticotropic hormone-releasing hormone in tissue of the intestinal tract of a subject, improving or enhancing the mechanical barrier of the intestinal tract of a subject, maintaining immune homeostasis in the intestinal tract of a subject, promoting the development of the intestinal tract of a subject and maintaining the energy homeostasis of cells of the intestinal tract of a subject, and improving energy metabolism in the intestinal tract, promoting the development and metabolism of muscle tissue of a subject, promoting the development and metabolism of adipose tissue of a subject, decreasing an allergic reaction in the body and intestinal tract of a subject, reducing the insulin resistance in a subject during pregnancy, controlling blood sugar in a subject during pregnancy, preventing gestational diabetes, preventing or improving gestational intrahepatic cholestasis in a subject, and preventing or treating a disease in a subject resulting from bacteria of the Helicobacter pylori genus.

## Description

### TECHNICAL FIELD

The present invention relates to substances and methods for improving the health and homeostasis of the intestinal tract, and the nutrition and development of a subject.

### BACKGROUND ART

Odd-carbon fatty acids (OCFAs) are a class of fatty acids having an odd number of carbon atoms. Natural lipids contain small amounts of OCFAs, which are a normal constituent part of the body of animals, and can be normally metabolized in the body. It is generally believed in the prior art that the accumulation of OCFAs in the human body does not affect the normal growth and development of the human body.

Some studies have shown that odd-carbon fatty acids have properties and physiological efficacies different from those of even-carbon fatty acids: compared with even-carbon fatty acids, odd-carbon fatty acids have a looser crystal structure and a weaker intermolecular force; odd-carbon fatty acids have metabolic pathways different from those of even-carbon fatty acids in the human body, and propionyl CoA generated by β-oxidation has a glycogenic effect and is a dynamic endogenous indicator for human body dietary assessment; pentadecanoic acid and heptadecanoic acid can be used to assess the risks of coronary heart disease and diabetes; pentadecanoic acid and nonadecanoic acid have strong inhibitory effects on the growth of various cancer cells, and so on and so forth.

### BRIEF DESCRIPTION OF THE INVENTION

The present invention aims to develop novel uses of odd-carbon fatty acids, or triglycerides containing odd-carbon fatty acids.

In particular, the present invention provides uses of odd-carbon fatty acids in the preparation of formulations or medicaments for any one, any two, any three, any four, any five, any six, any seven, any eight, any nine, any ten, any eleven, any twelve, any thirteen, any fourteen, any fifteen, any sixteen, any seventeen, any eighteen, any nineteen, any twenty, any twenty one, any twenty two, any twenty three, any twenty four, any twenty five, any twenty six, any twenty seven, any twenty eight, any twenty nine, any thirty, any thirty one, any thirty two, any thirty three, any thirty four, any thirty five, any thirty six, any thirty seven, any thirty eight, or all thirty nine of the thirty nine purposes listed herein.

The present invention further provides oils or fats for use in the above-identified uses.

More detailed content of the present invention is as described below.

### BRIEF DESCRIPTION OF THE DRAWINGS

Fig. 1: Differences in the expression levels of various tight junction-associated proteins in the duodenum, jejunum, and ileum of piglets ingesting different oil or fat dietary supplements.
Fig. 2: Differences in the content levels of CRF in the ileum of piglets ingesting different oil or fat dietary supplements.
Fig. 3: Differences in the content levels of IgG, IgA, and IgM in the jejunum of piglets ingesting different oil or fat dietary supplements.
Fig. 4: Differences in the content levels of IgG, IgA, and IgM in the ileum of piglets ingesting different oil or fat dietary supplements.
Fig. 5: Differences in the protein expression levels of TLR4 and NF-κB in the duodenum, jejunum, and ileum of piglets ingesting different oil or fat dietary supplements.
Fig. 6: Differences in the content levels of TNF-α, Il-1β, IL-6, and IL-10 in the ileum of piglets ingesting different oil or fat dietary supplements.
Fig. 7: Differences in the gene expression levels of MUC-2 in the ileum of piglets ingesting different oil or fat dietary supplements.
Fig. 8: Differences in the protein expression levels of Ki67 protein in the duodenum, jejunum, and ileum of piglets ingesting different oil or fat dietary supplements.
Fig. 9: Differences in the protein expression levels of p-mTOR (phosphorylated mTOR) in the duodenum, jejunum, and ileum of piglets ingesting different oil or fat dietary supplements.
Fig. 10: Differences in the protein expression levels of p-p70S6K (phosphorylated p70S6K) in the duodenum, jejunum, and ileum of piglets ingesting different oil or fat dietary supplements.
Fig. 11: Differences in the content levels of maltase in the duodenum and aminopeptidase N and sucrase in the ileum of piglets ingesting different oil or fat dietary supplements.
Fig. 12: Differences in the content levels of 5-hydroxytryptamine in the duodenum, jejunum, and ileum, and differences in the content levels of SP in the ileum, of piglets ingesting different oil or fat dietary supplements.
Fig. 13: Differences in the protein expression levels of key proteins of mitochondrial autophagy ULK1, TFEB, and Parkin in the duodenum and jejunum, and ULK1 and TFEB in ileum, of piglets ingesting different oil or fat dietary supplements.
Fig. 14: Differences in the protein expression levels of PPARγ and SREBP-1 in the duodenum and jejunum of piglets ingesting different oil or fat dietary supplements.
Fig. 15: Differences in the protein expression levels of MYHC1, MYHC2a, MYHC2b, MYHC2x, mTOR, p-AMPK, and PGC1-α in the dorsal muscle of piglets ingesting different oil or fat dietary supplements.
Fig. 16: Differences in the gene expression levels of SREBP-1C in the dorsal muscle of piglets ingesting different oil or fat dietary supplements.
Fig. 17: Differences in the protein expression levels of p-mTOR (phosphorylated mTOR), Prdm16 and Irisin in the dorsal fat, and p-mTOR and Prdm16 in the perirenal fat, of piglets ingesting different oil or fat dietary supplements.
Fig. 18: Differences in the gene expression levels of SREBP-1C and APOB in the dorsal fat of piglets ingesting different oil or fat dietary supplements.
Fig. 19: Differences in the content levels of serum glutathione peroxidase (GSH-PX) in piglets ingesting different oil or fat dietary supplements.
Fig. 20: Differences in the content levels of IgE and HIS in the serum, jejunum, and ileum of piglets ingesting different oil or fat dietary supplements.
Fig. 21: The average daily gain, average daily feed intake and feed conversion ratio in piglets ingesting different oil or fat dietary supplements during the experiment period (the average daily feed intake and feed conversion ratio of the breast milk group G0 could not be accurately statistically analyzed).
Fig. 22: The ratios of the weight of liver, brain, cerebellum, or brainstem to the body weight in piglets ingesting different oil or fat dietary supplements.
Fig. 23: The content levels of serum cortisol and adrenocorticotropic hormone ACTH in piglets ingesting different oil or fat dietary supplements.
Fig. 24: The content levels of serum HDL-C, LDL-C, ALT, AST, TG, and CHOL in piglets ingesting different oil or fat dietary supplements.
Fig. 25: The content levels of serum TP, ALB, BUN, and CREA in piglets ingesting different oil or fat dietary supplements.
Fig. 26: The content levels of serum TNF-α in piglets ingesting different oil or fat dietary supplements.
Fig. 27: The levels of serum IgG and IgM, and the content levels of spleen IgA, in piglets ingesting different oil or fat dietary supplements.
Fig. 28: The content levels of serum D-LACT in piglets ingesting different oil or fat dietary supplements.
Fig. 29: The content levels of fasting serum glucose (GLU) and fasting serum insulin (INS), and fasting insulin resistance index, in pregnant mice ingesting different oils or fats and daily rations.
Fig. 30: The content levels of serum bile acid in pregnant mice ingesting different oils or fats and daily rations.
Fig. 31: The content levels of superoxide dismutase (SOD), glutathione peroxidase (GSH-PX), and malondialdehyde (MDA) in the liver of pregnant mice ingesting different oils or fats and daily rations.
Fig. 32: The histological lesion scores of the colon, and HE staining images of the colon, of pregnant mice ingesting different oils or fats and daily rations.
Fig. 33: The gene expression levels of colonic inflammatory factors IL-1β, IL-6, TNF-α, NLRP3, and GSDMD in pregnant mice ingesting different oils or fats and daily rations.
Fig. 34: The levels of ratio of determined sequences of the Helicobacter pylori genus, and of the Deferribacter genus, in the intestinal tract of pregnant mice ingesting different oils or fats and daily rations.
Fig. 35: The feed conversion ratios in pregnant mice ingesting different oils or fats and daily rations during the experiment period.
Fig. 36: The content levels of serum TC, TG, HDL, and LDL in pregnant mice ingesting different oils or fats and daily rations.
Fig. 37: Differences in the protein expression levels of HIF-1α in the duodenum, jejunum, and ileum of piglets ingesting different oil or fat dietary supplements.
Fig. 38: Scanning electron microscopy images of the intestinal tracts of piglets ingesting different oil or fat dietary supplements.
Fig. 39: HE staining images of the intestinal tracts of piglets ingesting different oil or fat dietary supplements.
Fig. 40: The duodenal villus height, the duodenal crypt depth and the ratio of both in piglets ingesting different oil or fat dietary supplements.
Fig. 41: The jejunal villus height, the jejunal crypt depth and the ratio of both in piglets ingesting different oil or fat dietary supplements.
Fig. 42: The ileal villus height, the ileal crypt depth and the ratio of both in piglets ingesting different oil or fat dietary supplements.

In the above figures, * indicates P < 0.05, ** indicates P < 0.01, *** indicates P < 0.005, and ns indicates no significant difference.

### SPECIFIC EMBODIMENTS

It should be understood that, within the scope of the present invention, the above-identified technical features of the present invention and the technical features specifically described below (such as in the examples) can be combined with each other to form preferred technical solutions. Unless otherwise specified, the terms used herein have the same meanings as commonly understood by a person skilled in the art. Unless otherwise specified, the various percentage contents (X%) and the ratios between ingredients used in the present application are based on weight ratios.

In the present application, studies on the physiological and medical effects of odd-carbon fatty acids have been conducted, leading to findings of novel biological activities and functions of odd-carbon fatty acids. Therefore, the present application aims to provide novel uses of odd-carbon fatty acids.

As used herein, an "odd-carbon fatty acid" refers to a fatty acid having an odd number of carbon atoms, including but not limited to C11:0 fatty acid (undecanoic acid), C13:0 fatty acid (tridecanoic acid), C15:0 fatty acid (pentadecanoic acid), C17:0 fatty acid (heptadecanoic acid), C19:0 fatty acid (nonadecanoic acid), C21:0 fatty acid (heneicosanoic acid), C23:0 fatty acid (tricosanoic acid), or any combination of the foregoing. In some embodiments, the odd-carbon fatty acids described in the present application especially include one or more of C11:0 fatty acid (undecanoic acid), C13:0 fatty acid (tridecanoic acid), C15:0 fatty acid (pentadecanoic acid), and C17:0 fatty acid (heptadecanoic acid), or consists of one or more of C11:0 fatty acid (undecanoic acid), C13:0 fatty acid (tridecanoic acid), C15:0 fatty acid (pentadecanoic acid), and C17:0 fatty acid (heptadecanoic acid).

In some embodiments, in the uses described herein, the content of C15:0 fatty acid is 80% or more, preferably 85% or more, 90% or more, or 95% or more, based on the total amount of the odd-carbon fatty acids. In some embodiments, the content of C15:0 fatty acid is 99% or more based on the total amount of the odd-carbon fatty acids. In some embodiments, in the uses described herein, the odd-carbon fatty acids further comprise C17:0 in addition to C15:0; based on the total weight of the odd-carbon fatty acids, the content of C17:0 is ≤ 18%; in some embodiments, the content of C17:0 is ≤ 13%; in some embodiments, the content of C17:0 is ≤ 5%; in some embodiments, the content of C17:0 is ≤ 3%; and in some embodiments, the content of C17:0 is ≤ 1%.

It should be understood that in the uses of odd-carbon fatty acids described herein, the odd-carbon fatty acids may be used in the form of an odd-carbon fatty acid, or may be used in the form of a fatty acid chain contained in a glyceride such as a monoglyceride, a diglyceride and/or a triglyceride, In some embodiments, the triglyceride is an oil or fat. As used herein, the content percentage of a certain kind of fatty acid based on the total amount of the odd-carbon fatty acids has the following conventional meaning: (1) when present in the form of a fatty acid, the percentage of the fatty acid based on the total amount of the odd-carbon fatty acids; and (2) when present in the form of a glyceride, the percentage of the fatty acid residue based on the total amount of fatty acid residues attached to the glyceride. Unless otherwise specified, the content herein is expressed by weight or weight percentage.

Thus, in some embodiments, the present application provides uses of a glyceride, such as an oil or fat, containing odd-carbon fatty acids in the fatty acid composition. Preferably, in the fatty acid composition of the glyceride, especially the oil or fat, the content of the odd-carbon fatty acids is ≥ 20%, such as 20% to 70% or 20% to 60%. The odd-carbon fatty acid composition of the glyceride, especially the oil or fat, may include any one of the odd-carbon fatty acids described above, and preferably at least comprises one or more of C11:0 fatty acid (undecanoic acid), C13:0 fatty acid (tridecanoic acid), C15:0 fatty acid (pentadecanoic acid) and C17:0 fatty acid (heptadecanoic acid) or consists of one or more of C11:0 fatty acid (undecanoic acid), C13:0 fatty acid (tridecanoic acid), C15:0 fatty acid (pentadecanoic acid), and C17:0 fatty acid (heptadecanoic acid). Preferably, the odd-carbon fatty acids of the glyceride, especially the oil or fat, at least comprise C15:0 fatty acid (pentadecanoic acid).

In the odd-carbon fatty acid composition of the glyceride, especially the fat or oil, the content of C15:0 fatty acid is 80% or more, preferably 85% or more, 90% or more, or 95% or more, based on the total amount of the odd-carbon fatty acids. In some embodiments, the content of C15:0 fatty acid is 99% or more based on the total amount of the odd-carbon fatty acids. In some embodiments, the odd-carbon fatty acids further comprise C17:0 in addition to C15:0; based on the total weight of the odd-carbon fatty acids, the content of C17:0 is ≤ 18%; in some embodiments, the content of C17:0 is ≤ 13%; in some embodiments, the content of C17:0 is ≤ 5%; in some embodiments, the content of C17:0 is ≤ 3%; and in some embodiments, the content of C17:0 is ≤ 1%.

In some embodiments, in the fatty acid composition of the glyceride, especially the oil or fat: the content of C11:0 fatty acid is not higher than 5%, preferably not higher than 1%, and more preferably not higher than 0.5%; the content of C13:0 fatty acid is 1% to 5%, preferably 2% to 4%; C15:0 fatty acid is 15% to 70%, preferably 18% to 60%; the content of C17:0 fatty acid is 0.1% to 9%; the content of C19:0 fatty acid is not higher than 5%, preferably not higher than 1%, and more preferably not higher than 0.5%; the content of C21:0 fatty acid is not higher than 5%, preferably not higher than 1%, and more preferably not higher than 0.5%; and the content of C23:0 fatty acid is not higher than 5%, preferably not higher than 1%, and more preferably not higher than 0.5%.

In some embodiments, the fatty acid composition of the glyceride, especially the oil or fat, further comprises any one or more of C12:0, C14:0, C14:1, C16:0, C16:1, C18:0, C18:1, C18:2, C20:0, C18:3, C18:3T, C20:1, C20:2, C22:0, C20:3N6, C20:4N6, C20:5N3, C22:1, C20:3N3, C23:0, C22:2, C22:5, C22:6, C24:0, and C24:1. Preferably, in the fatty acid composition of the glyceride, especially the oil or fat, the content of C16:0 may be 2% to 15%, such as 4% to 13%; the content of C18:0 may be ≤ 5%, such as 0% to 5% or 0% to 3%; the content of C18:1 may be 5% to 35%, such as 7% to 33%; the content of C18:2 may be ≤ 35%, such as 0% to 35%; the content of C22:5 may be ≤ 5%, such as 0% to 5% or 1% to 3.5%; the content of C22:6 may be 5% to 25%, such as 8% to 22%; and the content of the remaining fatty acids is generally not more than 1% or less than 0.5%.

In some embodiments, in the fatty acid composition of the glyceride, especially the oil or fat, the content of C13:0 is 1% to 5%, preferably 2% to 4%, the content of C15:0 is 40% to 60%, preferably 45% to 53%, the content of C16:0 is 1% to 7%, preferably 3% to 5.5%, the content of C17:0 is 4% to 9%, preferably 5% to 9%, the content of C18:1 is 5% to 10%, preferably 6% to 8%, the content of C22:5 is 1% to 5%, preferably 2% to 4%, and the content of C22:6 is 15% to 25%, preferably 18% to 22%; and the content of the remaining fatty acids is generally less than 1% or less than 0.5%. In some embodiments, in the fatty acid composition of the glyceride, especially the oil or fat, the content of C15:0 is 40% to 65%, preferably 50% to 60%, the content of C16:0 is 1% to 7%, preferably 3% to 5.5%, the content of C17:0 is 0.1% to 3%, preferably 0.1% to 1%, the content of C18:0 is 1% to 5%, preferably 1% to 3%, the content of C18:1 is 25% to 35%, preferably 28% to 34%, and the content of C18:2 is 1% to 5%, preferably 2% to 4%; and the content of the remaining fatty acids is generally less than 1% or less than 0.5%.

In some embodiments, in the fatty acid composition of the glyceride, especially the oil or fat, the content of C15:0 is 12% to 25%, preferably 16% to 22%, the content of C16:0 is 5% to 15%, preferably 10% to 15%, the content of C17:0 is 1% to 7%, preferably 2% to 5%, the content of C18:0 is 0.5% to 3%, preferably 1% to 2%, the content of C18:1 is 12% to 22%, preferably 15% to 20%, the content of C18:2 is 25% to 35%, preferably 30% to 35%, and the content of C22:6 is 5% to 15%, preferably 7% to 12%; and the content of the remaining fatty acids is generally less than 1% or less than 0.5%.

It should be understood that the content of each fatty acid in the fatty acid composition described herein is determined with reference to the third method, i.e., "normalization method" in "GB5009.168-2016 Food Safety National Standard Determination of Fatty Acids in Foods".

In some embodiments, the oil or fat is derived from an edible oil (e.g., one or more edible oils), a transesterification product of the edible oil, or any combination of the foregoing. In some embodiments, the triglyceride containing odd-carbon fatty acids is prepared by transesterification with a conventional edible oil.

In some embodiments, the edible oil is selected from a vegetable oil, a microbial oil, an animal oil, or any combination of the foregoing; preferably, the vegetable oil includes one or more of rice oil, sunflower seed oil (such as high-oleic sunflower seed oil), rape oil, palm oil, palm kernel oil, peanut oil, rapeseed oil, soybean oil, cottonseed oil, safflower seed oil, perilla seed oil, tea seed oil, olive oil, cacao bean oil, Triadica sebifera seed oil, sweet almond oil, almond oil, Vernicia fordii seed oil, rubber seed oil, corn oil, wheat germ oil, sesame seed oil, castor seed oil, evening primrose seed oil, hazelnut oil, pumpkin seed oil, walnut oil, grape seed oil, borage seed oil, sea buckthorn seed oil, tomato seed oil, macadamia nut oil, coconut oil and cacao butter, or a fractionation product thereof, a self-transesterification product thereof or a transesterification product of two or more kinds of oil or fat; and the animal oil is selected from one or more of lard, chicken fat, sheep fat, fish oil and beef fat;
In some embodiments, the microorganism may be selected from: algae, such as strains of the order Thraustochytriales, the order Thraustochytriales more specifically including strains of the genus Thraustochytrium and the genus Schizochytrium; oleaginous bacteria, such as the genus Rhodococcus, e.g., Rhodococcus opacus; oleaginous yeasts, such as the genus Yarrowia, e.g., Yarrowia lipolytica; and mutant strains derived from any of the above-mentioned strains and mixed strains thereof, the microorganism preferably being a Schizochytrium sp.

Transesterification includes chemical transesterification and enzymatic transesterification. Chemical transesterification may be implemented using a chemical catalyst conventionally used for chemical transesterification. Exemplary chemical catalysts include, but are not limited to, acidic catalysts and basic catalysts. Preferably, the chemical catalyst is NaOH, KOH, NaOCH₃, sodium ethoxide, an organic base, a solid base catalyst, sulfuric acid, sulfonic acid, or a solid acid catalyst; more preferably, the chemical catalyst is NaOH, KOH, NaOCH₃, sulfuric acid, or sulfonic acid; and more preferably, the chemical catalyst is sodium methoxide, NaOH, and/or KOH. The usage amount of the chemical catalyst may be 0.1% to 1.0% of the total weight of the feedstock oil. The chemical catalyst may be used in a form well known in the art. For example sodium methoxide is typically added to the feedstock oil in the form of a solid, and NaOH and KOH are typically used in the form of an aqueous solution. An exemplary aqueous solution concentration may be 30% to 50%. The usage amount described herein refer to the usage amount of the catalyst per se, not the usage amount of the solution. The reaction may be carried out at a temperature of from 80°C to 150°C, preferably from 100°C to 120°C. The reaction can be carried out under reduced pressure (e.g., a vacuum degree of ≤ 10 mbar) for a period of time (e.g., 0.5 h to 1 h) such that fatty acids are uniformly distributed on the glycerol backbone. After completion of the transesterification, the reaction product is washed with water.

When enzymatic transesterification is employed, a lipase may be used as a catalyst. A lipase powder or an immobilized lipase in which the lipase is immobilized on a carrier (e.g., diatomaceous earth, ion exchange resin, etc.) may be used as the lipase. The lipase preferably used is a lipase lacking site specificity, including, but not limited to, a lipase derived from the genus Alcaligenes, a lipase derived from the genus Candida, etc. Suitable lipases include various commercially available immobilized enzymes or fermentation broths thereof. For example, Lipozyme TL IM, Lopozyme RM, etc., from Novozymes Corp., and immobilized enzymes or fermentation broths thereof from Amano Enzyme, Japan. The amount of lipase added is normally from one ten-thousandth to ten ten-thousandth of the weight of the feedstock oil. The reaction temperature for pre-transesterification may be in the range of from 40°C to 80°C, such as 70±5°C, depending on the optimum reaction temperature for the lipase used. Likewise, when the rate of transesterification reaches 0.1% to 1.0%, the pre-transesterification treatment is stopped. The reaction time of the enzymatic pre-transesterification treatment may be in the range of from 0.5 h to 10 h, such as 0.5 h to 3 h, depending on specific reaction conditions.

After completion of the transesterification, the resulting transesterified oil or fat may be subjected to conventional refining, such as decolorization and deodorization.

A glyceride containing odd-carbon fatty acids may be prepared by reacting the odd-carbon fatty acids with glycerol. For example, the odd-carbon fatty acids and glycerol may undergo an esterification reaction in the presence of a lipase. Suitable lipases may be lipases commonly used in the art for esterification reactions, such as the immobilized enzyme NOVO435 (Novozymes). Generally, the molar ratio of the odd-carbon fatty acids to glycerol may be 2 to 5:1. The odd-carbon fatty acids used in the esterification reaction may be a mixture of several kinds of odd-carbon fatty acids, or may be one kind of odd-carbon fatty acid. Generally, after mixing the odd-carbon fatty acids with glycerol, the mixture is heated to 65°C to 75°C, then the lipase is added in an amount of 1% to 20%, e.g., 5% to 15% by weight of the substrate, and the reaction is carried out under the condition of a vacuum degree of ≤ 10 mbar until completion of the reaction, thereby obtaining the glyceride containing odd-carbon fatty acids used in the present invention. The reaction may be carried out under stirring conditions. The obtained glyceride containing odd-carbon fatty acids may be purified by means of molecular distillation.

The edible oil used in the transesterification reaction may be a vegetable oil, a microbial oil or an animal oil well known in the art, including but not limited to: one or more of rice oil, sunflower seed oil (such as high-oleic sunflower seed oil), rape oil, palm oil, palm kernel oil, peanut oil, rapeseed oil, soybean oil, cottonseed oil, safflower seed oil, perilla seed oil, tea seed oil, olive oil, cacao bean oil, Triadica sebifera seed oil, sweet almond oil, almond oil, Vernicia fordii seed oil, rubber seed oil, corn oil, wheat germ oil, sesame seed oil, castor seed oil, evening primrose seed oil, hazelnut oil, pumpkin seed oil, walnut oil, grape seed oil, borage seed oil, sea buckthorn seed oil, tomato seed oil, macadimia nut oil, coconut oil and cacao butter. The animal oil is selected from one or more of lard, chicken fat, sheep fat, fish oil and beef fat. The microbial oil includes algae oil. The edible oil also includes transesterification products and fractionation products of various oils, including but not limited to various stearins obtained by fractionation, such as palm stearin. Suitable edible oil may be selected depending on other effects desired to be obtained. For example, an algae oil rich in DHA (docosahexaenoic acid) and/or a vegetable oil rich in oleic acid, such as high-oleic sunflower seed oil, may be selected. In some embodiments, the edible oil may further include palm stearin.

When carrying out transesterification, a suitable ratio of the usage amount of the triglyceride containing odd-carbon fatty acids to that of the vegetable oil can be selected according to the specific reaction conditions and the desired content of the odd-carbon fatty acids in the oil or fat. For example, in some embodiments, the weight ratio of the triglyceride containing odd-carbon fatty acids to the vegetable oil may be 45-70:30-55. In some embodiments, the weight ratio of the triglyceride containing odd-carbon fatty acids to the vegetable oil may be 50-65:35-50. In some embodiments, the weight ratio of the triglyceride containing odd-carbon fatty acids to the vegetable oil may be 55-62:38-45.

In some embodiments, the oil or fat is a chemical transesterification product of the triglyceride containing odd-carbon fatty acids with a DHA algae oil and a high-oleic sunflower seed oil; preferably, in the mixture of the triglyceride containing odd-carbon fatty acids with the DHA algae oil and the high-oleic sunflower seed oil, the weight ratio of triglyceride pentadecanoate to the DHA algae oil to the high-oleic sunflower seed oil is 40-50:30-45:1-10, such as 40-50:30-40:5-10 or 40-50:38-45:1-5. In some embodiments, in the fatty acid composition of the oil or fat, the content of C15:0 is 12% to 25%, preferably 16% to 22%, the content of C16:0 is 5% to 15%, preferably 10% to 15%, the content of C17:0 is 1% to 7%, preferably 2% to 5%, the content of C18:0 is 0.5% to 3%, preferably 1% to 2%, the content of C18:1 is 12% to 22%, preferably 15% to 20%, the content of C18:2 is 25% to 35%, preferably 30% to 35%, and the content of C22:6 is 5% to 15%, preferably 7% to 12%; and the content of the remaining fatty acids is generally less than 1% or less than 0.5%.

In some embodiments, the oil or fat is a chemical transesterification product of the triglyceride containing odd-carbon fatty acids with palm stearin and high-oleic sunflower seed oil; preferably, in a mixture of the triglyceride containing odd-carbon fatty acids with the palm stearin and the high-oleic sunflower seed oil, the weight ratio of triglyceride pentadecanoate to the palm stearin to the high-oleic sunflower seed oil is 55-62:1-5:35-40. In some embodiments, in the fatty acid composition of the oil or fat, the content of C15:0 is 40% to 65%, preferably 50% to 60%, the content of C16:0 is 1% to 7%, preferably 3% to 5.5%, the content of C17:0 is 0.1% to 3%, preferably 0.1% to 1%, the content of C18:0 is 1% to 5%, preferably 1% to 3%, the content of C18:1 is 25% to 35%, preferably 28% to 34%, and the content of C18:2 is 1% to 5%, preferably 2% to 4%; and the content of the remaining fatty acids is generally less than 1% or less than 0.5%.

The uses of odd-carbon fatty acids described herein include one or more of the following items of the odd-carbon fatty acids, and also include uses of the odd-carbon fatty acids in the preparation of a formulation or medicament for one or more of the following purposes:
(1) Increasing the protein expression abundance of a tight junction-associated protein (such as Occludin, Claudin and/or ZO-1) in a subject; especially, increasing the protein expression abundance of the tight junction-associated protein (e.g. Occludin, Claudin and/or ZO-1) in the intestinal tract (e.g. mucosa of the intestinal tract), and more specifically the protein expression abundance of the tight junction-associated protein (e.g. Occludin, Claudin and/or ZO-1) in the duodenum, jejunum, and ileum (e.g. mucosa thereof);
(2) Reducing the content of a corticotropin-releasing hormone (CRF) in a tissue of the intestinal tract of a subject; and more preferably, reducing the content of the corticotropin-releasing hormone (CRF) in a tissue of the ileum;
(3) Improving or enhancing the mechanical barrier of the intestinal tract of a subject; wherein improving the mechanical barrier of the intestinal tract can protect against the invasion of bacteria, toxins and foreign antigen substances to the body, maintain the stability of the body's internal environment, and create a basis for nutrient absorption;
(4) Increasing the content of an immunoglobulin (e.g., IgG, IgA and/or IgM) in the intestinal tract of a subject; preferably, increasing the content of an immunoglobulin (such as IgG, IgA, and/or IgM) in the jejunum and ileum; more preferably, increasing the content of immunoglobulin IgG, IgA, and/or IgM in the jejunum; and more preferably, increasing the content of immunoglobulin A in the ileum;
(5) Increasing the protein expression abundance of TLR4 and NF-κB in a tissue of the intestinal tract, especially in the duodenum, jejunum and ileum, of the subject;
(6) Maintaining the homeostasis of an ileal inflammatory cytokine (e.g., TNF-α, IL-1β, IL-6, and/or IL-10) in a subject;
(7) Increasing the expression amount of ileal mucin 2 in a subject;
(8) Increasing the expression of HIF-1α in the intestinal tract, and promoting the survival of cells of the intestinal tract in the hypoxic environment of the intestinal tract;
(9) Maintaining immune homeostasis in the intestinal tract (especially the ileum) of a subject;
(10) Increasing the content of the nuclear protein Ki-67 in a tissue of the intestinal tract of a subject, promoting the proliferation of intestinal epithelial cells in the subject, the tissue of the intestinal tract preferably being duodenum, jejunum and/or ileum;
(11) Activating the mTOR signaling pathway in the intestinal tract of a subject, increasing the expression amount of an activated mTOR protein in cells of the intestinal tract (especially cells of the small intestine, such as the duodenum, jejunum, and ileum) and/or increasing the level of an activated S6 kinase in cells of the intestinal tract (especially cells of the small intestine, such as the duodenum, jejunum, and ileum);
(12) Increasing the level of digestive enzymes (including but not limited to maltase, aminopeptidase and/or sucrase) in the intestinal tract, especially the small intestine, of a subject, and promoting the absorption of nutrients;
(13) Increasing the content of 5-hydroxytryptamine in a tissue of the intestinal tract (especially the duodenum, jejunum and/or ileum) of a subject and the content of Substance P (a neuropeptide) in the ileum, and enhancing the function of the small intestine such as increasing intestinal peristalsis, promoting the contraction of the smooth muscle of the gastrointestinal tract, inhibiting the secretion of gastric acid and bile, regulating local gastrointestinal blood flow, etc.;
(14) Promoting the development of the intestinal tract in a subject;
(15) Down-regulating the expression amount of a mitochondrial autophagy-associated protein (e.g., ULK1, TFEB, and/or Parkin) in cells of the intestinal tract (especially the duodenum, jejunum and/or ileum) of a subject, and alleviating mitochondrial dysfunction;
(16) Up-regulating the level of a lipid synthesis-associated transcription factor (such as PPARγ and/or SREBP-1) in the intestinal tract (especially the small intestine, and especially the duodenum and jejunum) of a subject, and increasing the absorption and anabolism of lipids in the intestinal tract (especially the small intestine, and especially the duodenum and jejunum);
(17) Maintaining the energy homeostasis of cells of the intestinal tract of a subject, and improving the energy metabolism in the intestinal tract;
(18) Up-regulating the content of type I and type II muscle fibers and the expression amount of an mTOR protein in muscle tissue of a subject, and at the same time reducing the relative content of AMPK and PGC1-α;
(19) Improving the energy state of muscle tissue of a subject, promoting the growth and development of cells, increasing the content of muscle fibers, inhibiting the conversion of type II muscle fibers into type I muscle fibers, increasing muscle mass, and promoting the overall development of muscle tissue;
(20) Up-regulating the expression amount of SREBP-1 in muscle tissue of a subject, promoting lipid anabolism, increasing the uptake and utilization of lipids by muscle tissue, improving energy state, and promoting the development and metabolism of muscle tissue;
(21) Promoting the development and metabolism of muscle tissue of a subject;
(22) Up-regulating the expression of an activated mTOR in a subject, reducing the expression amount of Prdm16 and Irisin, inhibiting lipolysis, and increasing lipid synthesis to promote the development of adipose tissue;
(23) Up-regulating the level of SERBP-1 and APOB in adipose tissue of a subject, increasing lipid synthesis in adipose tissue, enhancing transport of lipids by the body, and maintaining the energy balance of adipose tissue and the body;
(24) Promoting the development and metabolism of adipose tissue in a subject;
(25) Up-regulating the content of GSH-PX in serum of a subject, improving the overall antioxidant capacity of the body, and maintaining overall redox equilibrium;
(26) Reducing the content of blood HIS, jejunal IgE, ileal IgE, jejunal HIS and ileal HIS in a subject, and reducing an allergic reaction in the body, and the jejunum and ileum;
(27) Reducing an allergic reaction in the body and intestinal tract of a subject;
(28) Reducing insulin resistance in a subject during pregnancy;
(29) Controlling blood sugar in a subject during pregnancy;
(30) Improving insulin resistance resulting from a poor diet (e.g., a low-protein diet);
(31) Preventing gestational diabetes, and preventing fetal macrosomia, fetal malformation, premature birth, premature rupture of fetal membrane and/or polyhydramnios resulting from gestational diabetes, reducing fetal death rate;
(32) Reducing the level of serum bile acids in a subject during pregnancy;
(33) Preventing or improving gestational intrahepatic cholestasis in a subject, such as gestational intrahepatic cholestasis resulting from poor diet or disease; and reducing the occurrence or risk of occurrence of fetal distress, premature labor, and perinatal mortality rate associated with gestational intrahepatic cholestasis in the subject;
(34) Increasing the level of superoxide dismutase and glutathione peroxidase in the liver of a subject, reducing the level of malondialdehyde in the liver of the subject, and reducing oxidative stress in the subject during pregnancy;
(35) Improving the pathological damaging effect on a tissue of the intestinal tract induced by abnormal diet or disease in a subject;
(36) Reducing a colonic inflammatory factor (such as IL-1β, IL-6, TNF-α, NLRP3, and/or GSDMD) in a subject, and alleviating inflammation in the intestinal tract;
(37) Treating or preventing inflammatory bowel disease and colorectal cancer;
(38) Reducing the abundance of the Helicobacter pylori genus and/or Deferribacter genus in the body of a subject, and improving the microecology in the intestinal tract; and
(39) Preventing or treating a disease resulting from a bacterium of the Helicobacter pylori genus in a subject, including gastric cancer.

In some embodiments, the uses of odd-carbon fatty acids described herein include the following uses of the odd-carbon fatty acids, and also include uses of the odd-carbon fatty acids in the preparation of a formulation or medicament for the following purposes:
(1) Increasing the protein expression abundance of a tight junction-associated protein (such as Occludin, Claudin and/or ZO-1) in a subject; especially, increasing the protein expression abundance of the tight junction-associated protein (e.g. Occludin, Claudin and/or ZO-1) in the intestinal tract (e.g. mucosa of the intestinal tract), and more specifically the protein expression abundance of the tight junction-associated protein (e.g. Occludin, Claudin and/or ZO-1) in the duodenum, jejunum, and ileum (e.g. mucosa thereof); and
(2) Reducing the content of a corticotropin-releasing hormone (CRF) in tissue of the intestinal tract of the subject;

Thereby improving or enhancing the mechanical barrier of the intestinal tract of the subject.

In some embodiments, the uses of odd-carbon fatty acids described herein include the following uses of the odd-carbon fatty acids, and also include uses of the odd-carbon fatty acids in the preparation of a formulation or medicament for one or more of the following purposes:
(4) Increasing the content of an immunoglobulin (e.g., IgG, IgA, and/or IgM) in the intestinal tract of a subject; preferably, increasing the content of an immunoglobulin (such as IgG, IgA, and/or IgM) in the jejunum and ileum; and more preferably, increasing the content of immunoglobulin IgG, IgA, and/or IgM in the jejunum, and increasing the content of immunoglobulin A in the ileum;
(5) Increasing the protein expression abundance of TLR4 and NF-κB in a tissue of the intestinal tract, especially in the duodenum, jejunum and ileum, of a subject;
(6) Maintaining the homeostasis of an ileal inflammatory cytokine (e.g., TNF-α, IL-1β, IL-6, and/or IL-10) in a subject;
(7) Increasing the expression amount of ileal mucin 2 in a subject; and
(8) Increasing the expression of HIF-1α in the intestinal tract, and promoting the survival of cells of the intestinal tract in the hypoxic environment of the intestinal tract;
(9) Maintaining immune homeostasis in the intestinal tract (especially ileum) of the subject;

In some embodiments, the uses of odd-carbon fatty acids described herein include the following uses of the odd-carbon fatty acids, and also include uses of the odd-carbon fatty acids in the preparation of a formulation or medicament for one or more of the following purposes:
(10) Increasing the content of the nuclear protein Ki-67 in a tissue of the intestinal tract of a subject, and promoting the proliferation of intestinal epithelial cells of the subject; the tissue of the intestinal tract preferably being the duodenum, jejunum and/or ileum;
(11) Activating the mTOR signaling pathway in the intestinal tract of a subject, increasing the expression amount of an activated mTOR protein in cells of the intestinal tract (especially cells of the small intestine) and/or increasing the level of an activated S6 kinase in cells of the intestinal tract (especially cells of the small intestine);
(12) Increasing the level of digestive enzymes (including but not limited to maltase, aminopeptidase and/or sucrase) in the intestinal tract, especially the small intestine, of a subject, and promoting the absorption of nutrients; and
(13) Increasing the content of 5-hydroxytryptamine in a tissue of the intestinal tract (especially the duodenum, jejunum and/or ileum) of a subject and the content of Substance P in the ileum, enhancing the function of the small intestine such as increasing intestinal peristalsis, promoting the contraction of the smooth muscle of the gastrointestinal tract, inhibiting the secretion of gastric acid and bile, regulating local gastrointestinal blood flow, etc.;

Thereby promoting the development of the intestinal tract of the subject.

In some embodiments, the uses of odd-carbon fatty acids described herein include the following uses of the odd-carbon fatty acids, and also include uses of the odd-carbon fatty acids in the preparation of a formulation or medicament for one or more of the following purposes:
(15) Down-regulating the expression amount of a mitochondrial autophagy-associated protein (e.g., ULK1, TFEB, and/or Parkin) in cells of the intestinal tract of a subject, and alleviating mitochondrial dysfunction; and/or
(16) Up-regulating the level of a lipid synthesis-associated transcription factor (such as PPARγ and/or SREBP-1) in the intestinal tract (especially the small intestine) of a subject, and increasing the absorption and anabolism of lipids in the intestinal tract (especially the small intestine, and especially the duodenum and jejunum);

Thereby maintaining the energy homeostasis of cells of the intestinal tract of the subject, and improving the energy metabolism in the intestinal tract.

In some embodiments, the uses of odd-carbon fatty acids described herein include the following uses of the odd-carbon fatty acids, and also include uses of the odd-carbon fatty acids in the preparation of a formulation or medicament for one or more of the following purposes:
(18) Up-regulating the content of type I and type II muscle fibers and the expression amount of an mTOR protein in muscle tissue of a subject, and at the same time reducing the relative content of AMPK and PGC1-α; and/or
(19) Improving the energy state of muscle tissue of a subject, promoting cell growth and tissue development, increasing the content of muscle fibers, inhibiting the conversion of type II muscle fibers into type I muscle fibers, increasing muscle mass, and promoting the overall development of muscle tissue; and/or
(20) Up-regulating the expression amount of SREBP-1 in muscle tissue of a subject, promoting lipid anabolism, increasing the uptake and utilization of lipids by muscle tissue, improving energy state, and promoting the development and metabolism of muscle tissue;
thereby promoting the development and metabolism of muscle tissue of the subject.

In some embodiments, the uses of odd-carbon fatty acids described herein include the following uses of the odd-carbon fatty acids, and also include uses of the odd-carbon fatty acids in the preparation of a formulation or medicament for one or more of the following purposes:
(22) Up-regulating the expression of an activated mTOR in a subject, reducing the expression amount of Prdm16 and Irisin, inhibiting lipolysis, and increasing lipid synthesis to promote the development of adipose tissue; and/or
(23) Up-regulating the level of SERBP-1 and APOB in adipose tissue of a subject, increasing lipid synthesis in adipose tissue, enhancing transport of lipids by the body, and maintaining the energy balance of adipose tissue and the body;

Thereby promoting the development and metabolism of adipose tissue in the subject.

In some embodiments, the uses of odd-carbon fatty acids described herein include the following uses of the odd-carbon fatty acids, and also include uses of the odd-carbon fatty acids in the preparation of a formulation or medicament for one or more of the following purposes:
(25) Up-regulating the content of GSH-PX in serum of a subject, improving the overall antioxidant capacity of the body, and maintaining overall redox equilibrium; and/or
(26) Reducing the content of blood HIS, jejunal IgE, ileal IgE, jejunal HIS and ileal HIS in a subject, and reducing an allergic reaction in the body, and the jejunum and ileum;

Thereby reducing an allergic reaction in the body and intestinal tract of the subject.

In some embodiments, the uses of odd-carbon fatty acids described herein include the following uses of the odd-carbon fatty acids, and also include uses of the odd-carbon fatty acids in the preparation of a formulation or medicament for one or more of the following purposes:
(28) Reducing insulin resistance in a subject during pregnancy; and/or
(29) Controlling blood sugar in a subject during pregnancy;

Thereby preventing gestational diabetes, and preventing fetal macrosomia, fetal malformation, premature birth, premature rupture of fetal membrane, and/or polyhydramnios resulting from gestational diabetes, reducing fetal death rate.

In some embodiments, the uses of odd-carbon fatty acids described herein include the following uses of the odd-carbon fatty acids, and also include uses of the odd-carbon fatty acids in the preparation of a formulation or medicament for the following purposes: reducing the level of serum bile acids in a subject during pregnancy, thereby preventing or improving gestational intrahepatic cholestasis in the subject, such as gestational intrahepatic cholestasis resulting from a poor diet or a disease. In some embodiments, the uses of odd-carbon fatty acids described herein include the following uses of the odd-carbon fatty acids, and also include uses of the odd-carbon fatty acids in the preparation of a formulation or medicament for the following purposes: reducing the occurrence or risk of occurrence of fetal distress, premature labor, and perinatal mortality rate in a subject with gestational intrahepatic cholestasis.

In some embodiments, the uses of odd-carbon fatty acids described herein include the following uses of the odd-carbon fatty acids, and also include uses of the odd-carbon fatty acids in the preparation of a formulation or medicament for the following purposes: increasing the level of superoxide dismutase and glutathione peroxidase in the liver of a subject, reducing the level of malondialdehyde in the liver of the subject, and reducing oxidative stress in the subject during pregnancy, thereby improving the pathological damaging effect on a tissue of the intestinal tract induced by abnormal diet or disease in the subject.

In some embodiments, the uses of odd-carbon fatty acids described herein include the following uses of the odd-carbon fatty acids, and also include uses of the odd-carbon fatty acids in the preparation of a formulation or medicament for the following purposes: reducing a colonic inflammatory factor in a subject, thereby treating or preventing inflammatory bowel disease and colorectal cancer.

In some embodiments, the uses of odd-carbon fatty acids described herein include the following uses of the odd-carbon fatty acids, and also include uses of the odd-carbon fatty acids in the preparation of a formulation or medicament for the following purposes: reducing the Helicobacter pylori genus in the body of a subject, thereby preventing or treating a disease resulting from a bacterium of the Helicobacter pylori genus in the subject, including gastric cancer.

In some embodiments, the uses of odd-carbon fatty acids described herein include the following uses of the odd-carbon fatty acids, and also include uses of the odd-carbon fatty acids in the preparation of a formulation or medicament for the following purposes:
Improving or enhancing the mechanical barrier of the intestinal tract of a subject, maintaining the immune homeostasis in the intestinal tract (especially the ileum) of the subject, promoting the development of the intestinal tract of the subject and maintaining the energy homeostasis of cells of the intestinal tract of the subject, and improving the energy metabolism in the intestinal tract; and/or
Promoting the development and metabolism of muscle tissue in the subject; and/or
Promoting the development and metabolism of adipose tissue in the subject; and/or
Reducing an allergic reaction in the body and intestinal tract of the subject.

In some embodiments, the uses of odd-carbon fatty acids described herein include the following uses of the odd-carbon fatty acids, and also include uses of the odd-carbon fatty acids in the preparation of a formulation or medicament for the following purposes:
Increasing the health level of the intestinal tract, increasing the health level of the intestinal tract preferably being enhancing the mechanical barrier function of the intestinal tract, enhancing the immune homeostasis in the intestinal tract, promoting the development of the intestinal tract, increasing the digestive enzyme level in the intestinal tract, enhancing nutrient absorption, maintaining the energy homeostasis of cells of the intestinal tract, improving pathological damage to a tissue of the intestinal tract, alleviating inflammation in the intestinal tract, and/or improving the microecology in the intestinal tract; and/or
Improving the development and metabolism of muscle tissue; and/or
Improving the development and metabolism of adipose tissue; and/or
Increasing antioxidant capacity or reducing oxidative stress; and/or
Reducing an allergic reaction, preferably reducing an allergic reaction in the intestinal tract; and/or
Improving insulin resistance resulting from a low-protein diet; and/or
Improving bile acid metabolism.

In some embodiments, the uses of odd-carbon fatty acids described herein include the following uses of the odd-carbon fatty acids, and also include uses of the odd-carbon fatty acids in the preparation of a prophylactic agent, therapeutic agent or auxiliary therapeutic agent for at least one of the following diseases or conditions: inflammatory bowel disease, colorectal cancer, diarrhoea, irritable bowel syndrome, ulcers in the intestinal tract, allergies in the intestinal tract, dysbacteriosis in the intestinal tract, Helicobacter pylori infection, gestational diabetes (particularly for gestational diabetes patients with a low-protein diet or accompanied with a low-protein diet) and intrahepatic cholestasis (particularly gestational intrahepatic cholestasis).

In some embodiments, also provided herein are the following methods:
(1) A method for increasing the protein expression abundance of a tight junction-associated protein (such as Occludin, Claudin and/or ZO-1) in a subject; especially a method for increasing the protein expression abundance of the tight junction-associated protein (e.g. Occludin, Claudin and/or ZO-1) in the intestinal tract (e.g. mucosa of the intestinal tract), and more specifically a method for increasing the protein expression abundance of the tight junction-associated protein (e.g. Occludin, Claudin and/or ZO-1) in the duodenum, jejunum, and ileum (e.g. mucosa thereof);
(2) A method for reducing the content of a corticotropin-releasing hormone (CRF) in a tissue of the intestinal tract of a subject; and more preferably, a method for reducing the content of a corticotropin-releasing hormone (CRF) in a tissue of the ileum;
(3) A method for improving or enhancing the mechanical barrier of the intestinal tract of a subject;
(4) A method for increasing the content of an immunoglobulin (e.g., IgG, IgA and/or IgM) in the intestinal tract of a subject; preferably, a method for increasing the content of an immunoglobulin (such as IgG, IgA and/or IgM) in the jejunum and ileum; more preferably, a method for increasing the content of immunoglobulin IgG, IgA and/or IgM in the jejunum; and more preferably, a method for increasing the content of immunoglobulin A in the ileum;
(5) A method for increasing the protein expression abundance of TLR4 and NF-κ B in a tissue of the intestinal tract, especially in the duodenum, jejunum and ileum, of a subject;
(6) A method for maintaining the homeostasis of an ileal inflammatory cytokine (e.g., TNF-α, IL-1β, IL-6, and/or IL-10) in a subject;
(7) A method for increasing the expression amount of ileal mucin 2 in a subject;
(8) A method for increasing the expression of HIF-1α in the intestinal tract, and promoting the survival of cells of the intestinal tract in the hypoxic environment of the intestinal tract;
(9) A method for maintaining immune homeostasis in the intestinal tract (especially ileum) of a subject;
(10) A method for increasing the content of the nuclear protein Ki-67 in a tissue of the intestinal tract of a subject, the tissue of the intestinal tract preferably being the duodenum, jejunum and/or ileum; and a method for promoting the proliferation of intestinal epithelial cells in the subject;
(11) A method for activating the mTOR signaling pathway in the intestinal tract of a subject, and a method for increasing the expression amount of an activated mTOR protein in cells of the intestinal tract (especially cells of the small intestine, such as the duodenum, jejunum, and ileum) and/or increasing the level of an activated S6 kinase in cells of the intestinal tract (especially cells of the small intestine, such as the duodenum, jejunum, and ileum);
(12) A method for increasing the level of digestive enzymes (including but not limited to maltase, aminopeptidase and/or sucrase) in the intestinal tract, especially the small intestine, of a subject, and promoting the absorption of nutrients;
(13) a method for increasing the content of 5-hydroxytryptamine in a tissue of the intestinal tract (especially the duodenum, jejunum and/or ileum) of a subject and the content of Substance P (a neuropeptide) in the ileum, and a method for enhancing the function of the small intestine such as increasing intestinal peristalsis, promoting the contraction of the smooth muscle of the gastrointestinal tract, inhibiting the secretion of gastric acid and bile, regulating local gastrointestinal blood flow, etc.;
(14) A method for promoting the development of the intestinal tract in a subject;
(15) A method for down-regulating the expression amount of a mitochondrial autophagy-associated protein (e.g., ULK1, TFEB, and/or Parkin) in cells of the intestinal tract (especially the duodenum, jejunum and/or ileum) of a subject, and alleviating mitochondrial dysfunction;
(16) A method for up-regulating the level of a lipid synthesis-associated transcription factor (such as PPARγ and/or SREBP-1) in the intestinal tract (especially the small intestine, and especially the duodenum and jejunum) of a subject, and increasing the absorption and anabolism of intestinal lipids in the intestinal tract (especially the small intestine, and especially the duodenum and jejunum);
(17) A method for maintaining the energy homeostasis of cells of the intestinal tract of a subject, and improving energy metabolism in the intestinal tract;
(18) A method for up-regulating the content of type I and type II muscle fibers and the expression amount of an mTOR protein in muscle tissue of a subject, and at the same time reducing the relative content of AMPK and PGC1-α;
(19) A method for improving the energy state of muscle tissue of a subject, promoting the growth and development of cells, increasing the content of muscle fibers, inhibiting the conversion of type II muscle fibers into type I muscle fibers, increasing muscle mass, and promoting the overall development of muscle tissue;
(20) A method for up-regulating the expression amount of SREBP-1 in muscle tissue of a subject, promoting lipid anabolism, increasing the uptake and utilization of lipids by muscle tissue, improving energy state, and promoting the development and metabolism of muscle tissue;
(21) A method for promoting the development and metabolism of muscle tissue in a subject;
(22) A method for up-regulating the expression of an activated mTOR in a subject, reducing the expression amount of Prdm16 and Irisin, inhibiting lipolysis, and increasing lipid synthesis to promote the development of adipose tissue;
(23) A method for up-regulating the level of SERBP-1 and APOB in adipose tissue of a subject, increasing lipid synthesis in adipose tissue, enhancing transport of lipids by the body, and maintaining the energy balance of adipose tissue and the body;
(24) A method for promoting the development and metabolism of adipose tissue in a subject;
(25) A method for up-regulating the content of GSH-PX in serum of a subject, improving the overall antioxidant capacity of the body, and maintaining overall redox equilibrium;
(26) A method for reducing the content of blood HIS, jejunal IgE, ileal IgE, jejunal HIS, and ileal HIS in a subject, and reducing an allergic reaction in the body, and the jejunum and ileum;
(27) A method for reducing an allergic reaction in the body and intestinal tract of a subject;
(28) A method for reducing insulin resistance in a subject during pregnancy;
(29) A method for controlling blood sugar in a subject during pregnancy;
(30) A method for improving insulin resistance resulting from a poor diet (e.g., a low-protein diet);
(31) A method for preventing gestational diabetes, and a method for preventing fetal macrosomia, fetal malformation, premature birth, premature rupture of fetal membrane and/or polyhydramnios resulting from gestational diabetes, reducing fetal death rate;
(32) A method for reducing the level of serum bile acids in a subject during pregnancy;
(33) A method for preventing or improving gestational intrahepatic cholestasis in a subject, such as gestational intrahepatic cholestasis resulting from poor diet or disease; and a method for reducing the occurrence or risk of occurrence of fetal distress, premature labor, and perinatal mortality rate in subject with gestational intrahepatic cholestasis;
(34) A method for increasing the level of superoxide dismutase and glutathione peroxidase in the liver of a subject, reducing the level of malondialdehyde in the liver of the subject, and reducing oxidative stress in the subject during pregnancy;
(35) A method for improving the pathological damaging effect on a tissue of the intestinal tract induced by abnormal diet or disease in a subject;
(36) A method for reducing a colonic inflammatory factor (such as IL-1β, IL-6, TNF-α, NLRP3 and/or GSDMD) in a subject, and alleviating inflammation in the intestinal tract;
(37) A method for treating or preventing inflammatory bowel disease and colorectal cancer;
(38) A method for reducing the abundance of the Helicobacter pylori genus and/or Deferribacter genus in the body of a subject, and improving the microecology in the intestinal tract; and
(39) A method for preventing or treating a disease resulting from a bacterium of the Helicobacter pylori genus in a subject, including gastric cancer;

Each of the methods comprises administering to the subject in need thereof an effective amount of the odd-carbon fatty acids. The odd-carbon fatty acids are as described in any embodiment herein. It should be understood that the odd-carbon fatty acids may be provided in the form of an odd-carbon fatty acid in free form, or may be provided in the form of a glyceride (such as a monoglyceride, a diglyceride and/or a triglyceride) containing the odd-carbon fatty acids, or further may be provided in the form of an oil or fat (such as an oil or fat described herein).

In some embodiments of the uses and methods described herein, the oil or fat has the following fatty acid composition: the content of C15:0 is 50% to 60%, preferably 55% to 60%, the content of C16:0 is 1% to 7%, preferably 3% to 5.5%, the content of C17:0 is 0.1% to 3%, preferably 0.1% to 1%, the content of C18:0 is 1% to 5%, preferably 1% to 3%, the content of C18:1 is 25% to 35%, preferably 28% to 34%, and the content of C18:2 is 1% to 5%, preferably 2% to 4%; and the content of the remaining fatty acids is generally less than 1% or less than 0.5%. In some embodiments, the oil or fat is a chemical transesterification product of the triglyceride containing odd-carbon fatty acids with palm stearin and high-oleic sunflower seed oil; preferably, in a mixture of the triglyceride containing odd-carbon fatty acids with the palm stearin and the high-oleic sunflower seed oil, the weight ratio of triglyceride pentadecanoate to the palm stearin to the high-oleic sunflower seed oil is 55-62:1-5:35-40. Preferably, the uses and methods include one or more of the following items: increasing the protein expression abundance of a tight junction-associated protein (such as Occludin, Claudin and ZO-1) in the mucous membrane of the duodenum, jejunum, and ileum; reducing the content of CRF in a tissue of the intestinal tract; enhancing or improving the mechanical barrier of the intestinal tract of a piglet; increasing the levels of IgG, IgA, and IgM in the jejunum; increasing the level of IgA in the ileum; up-regulating the protein expression abundance of TLR4 and NF-κB in the duodenum, jejunum, and ileum; modulating an inflammatory cytokine (such as TNF-α, IL-1β, IL-6 and/or IL-10) to maintain the immune homeostasis in the ileum; maintaining the level of mucin in the mucous membrane of the intestinal tract and maintaining the immune homeostasis of the ileum; increasing the protein expression abundance of Ki67 in intestinal epithelial cells, promoting the proliferation of intestinal epithelial cells, and improving the mechanical barrier of the intestinal tract and promoting the development of the intestinal tract; activating the mTOR signaling pathway, increasing the expression amount of an activated mTOR protein in cells of the small intestine, and promoting cell growth and tissue development; increasing the level of an activated S6 kinase in cells of the small intestine, rescuing cell proliferation and protein metabolism in a tissue of the small intestine, and promoting tissue development; increasing the level of digestive enzymes (including maltase, aminopeptidase and/or sucrase) in the small intestine, and promoting the absorption of nutrients; increasing the content of 5-hydroxytryptamine in the duodenum, jejunum and ileum, and the content of Substance P in the ileum, regulating intestinal peristalsis, promoting the contraction of the smooth muscle of the gastrointestinal tract, inhibiting the secretion of gastric acid and bile, regulating local gastrointestinal blood flow, and enhancing the function of the small intestine; down-regulating the expression amount of an intracellular mitochondrial autophagy-associated protein (e.g., ULK1, TFEB, and/or Parkin), alleviating mitochondrial dysfunction, and maintaining energy homeostasis of cells of the intestinal tract; up-regulating the level of a lipid synthesis-associated transcription factor (including PPARγ and/or SREBP-1), improving the absorption and anabolism of lipids in the small intestine, and maintaining energy homeostasis of cells of the intestinal tract; up-regulating the content of type I and type II muscle fibers in muscle tissue and the expression amount of an mTOR protein, reducing the relative content of AMPK and PGC1-a, alleviating the energy state of muscle tissue, promoting cell proliferation and tissue development, increasing the content of muscle fibers, inhibiting the conversion of type II muscle fibers into type I muscle fibers, increasing muscle mass, and promoting the overall development of muscle tissue; up-regulating the expression amount of SREBP-1 in muscle tissue, increasing the uptake and utilization of lipids by muscle tissue, improving energy state, and promoting the development of muscle tissue; up-regulating the expression of an activated mTOR and reducing the expression amount of Prdm16 and Irisin, thereby inhibiting lipolysis, increasing lipid synthesis, and promoting the development of adipose tissue; up-regulating the level of SERBP-1 in adipose tissue, maintaining the level of APOB, increasing lipid synthesis in adipose tissue, enhancing transport of lipids by the body, and maintaining the energy balance of adipose tissue and the body; maintaining the content of GSH-PX in serum, improving the overall antioxidant capacity of the body, and maintaining overall redox equilibrium; reducing blood HIS, jejunal IgE, ileal IgE and HIS, and reducing an allergic reaction in the body and the jejunum and ileum.

As used herein, "individual" or "subject" mainly refers to mammals, such as humans, farmed livestock (e.g., pigs, cows, sheep, horses, camels, donkeys, etc.), research mammals (e.g., rats, mice, apes, monkeys, dogs, etc.), human-raised animals or wild animals, etc. The present application does not exclude individuals other than mammals, such as poultry, aquatic life, etc.

As used herein, an effective amount refers to an amount that, upon administration over a period of time, achieves an intended purpose. A person skilled in the art can determine the effective amount according to different individuals and different purposes or symptoms by using conventional methods in the art.

The formulation described herein may be a food product or, for example, a dietary supplement. The dietary supplement may contain other ingredients such as dietary cellulose, vitamins, minerals, etc., which are contained in conventional dietary supplements. The dietary supplement may be provided in the form of, for example, a capsule, a granule, a powder, a tablet, a drop or a chunk, or a capsule, or a pellet, or a semi-emulsion or emulsion, among others. The food product may be a beverage, a dairy product, a confection, a chocolate, a pastry, a snack food, a meat product, an egg product, a cold food, a pet food, or a feed. The dairy product may be milk powder, liquid milk, milk tablets, yogurt, etc. The pastry may be a biscuit, bread, cake, etc. The beverage may be a carbonated beverage, soda water, fruit flavored water, plum drink, a bulk low-sugar beverage, a mineral beverage, malted milk powder, milk tea, coffee, etc. The cold food may be an ice pop, a frozen dessert, an ice cream, a pudding, etc.

The medicament described herein may, in addition to containing the odd-carbon fatty acids described herein, further contain a pharmaceutically acceptable carrier well known in the art.

The present invention will be further described below in conjunction with specific examples. It should be understood that these examples are only used to illustrate the present invention, and are not intended to limit the scope of the present invention. The experimental methods for which specific conditions are not specified in the following examples are generally performed according to conventional conditions or manufacturers' recommended conditions. Unless otherwise specified, percentages and parts are by weight.

### Example 1

### 1. Basic experimental procedure

After mixing the oil or fat in the following table, chemical transesterification was performed such that fatty acids were uniformly distributed on the glycerol backbone, so as to eliminate effects on digestion, absorption and function resulting from differences in the position of the fatty acids. The resulting oil or fat, after being refined, was prepared into an oil-in-water emulsion. The oil-in-water emulsion was spray-dried to obtain an oil or fat powder. The oil or fat powder was fed to piglets as a dietary supplement.

| | |
|---|---|
| Oil or Fat 1 | Hard ST: high oleic sunflower seed oil = 73:27 |
| Oil or Fat 2 | OCFA algae oil: high oleic sunflower seed oil = 92:8 |
| Oil or Fat 3 | C15-TAG: Hard ST: high oleic sunflower seed oil = 59:4:37 |

The method for feeding the piglets is as follows. The piglets were weaned at 7 days of age, and were fed with commercial formula milk powder for 6 days. Then, every day, the first meal of liquid milk for the piglets had added thereto the oil or fat powder, and was fed to the piglets after the oil or fat powder dissolved. At other times, the piglets were fed normally with the commercial formula milk powder. Feeding was performed for a total of 15 days.

### 2. Preparation of triglyceride containing odd-carbon fatty acids

Glycerol (Yihai Kerry) and pentadecanoic acid (purchased from Titan Platform, Adamas, purity ≥ 95%) were mixed in a molar ratio of 1:4, heated to 70°C, and mixed uniformly as a reaction substrate. Immobilized enzyme NOVO435 (Novozymes) was added in an amount of 10% by weight of the substrate, and reaction was carried out under conditions of 70°C, 300 rpm and a vacuum degree of ≤ 10 mbar for 15 h, to obtain a crude oil or fat product. The obtained crude oil or fat product was subjected to molecular distillation separation. The heavy phase was glyceryl pentadecanoate (C15-TAG) with a purity of > 98%.

Using the above method, undecanoic acid (Adamas, purity ≥ 99%), tridecanoic acid (Accela, purity ≥ 95%) and heptadecanoic acid (Adamas, purity ≥ 98%) were used to prepare glyceryl tri-undecanoate, glyceryl tri-tridecanoate and glyceryl tri-heptadecanoate respectively.

### 3. Preparation of oil or fat

### Oil or Fat 1

Hard ST (Yihai Kerry, batch RBDHard ST 321, acid value 0.04 mg/KOH/g) and high-oleic sunflower seed oil (Yihai Kerry, model finished product first class) were melted and mixed uniformly in a mass ratio of 73:27. Then chemical transesterification was performed such that fatty acids were uniformly distributed on the glycerol backbone. The procedure of chemical transesterification was as follows: Dehydration was first performed under vacuum at 110°C for 1 h, then sodium methoxide was added in an amount of 0.2% by weight of the oil, and reaction was carried out at 110°C while vacuumizing for 1 h. The temperature was decreased to 80°C or lower, and citric acid (prepared into a 10% citric acid solution) was added in an amount of 1.8 times the mass of sodium methoxide. After stirring, the mixture was poured into a separatory funnel, washed with hot water until neutrality, and then dehydrated under vacuum at 110°C. The oil or fat obtained as described above could be refined according to a conventional oil or fat refining method. In this example, the following oil or fat refining method was used to perform refining to obtain Oil or Fat 1.

Oil or fat refining method:
1. 500 g of the oil or fat was weighed and added into a 2 L glass jacketed reactor, naturally heated until melting, and held at 60°C. The stirring speed was 200 rpm, and the whole process was protected by charging nitrogen. AV (acid value) and PV (peroxide value) were determined.
2. Washing with water was performed. A citric acid solution was added dropwise (the amount of water added was 15%, and the amount of citric acid added was 0.5 %o), and stirring was performed at 200 rpm for 5 min. The stirring was stopped, and the mixture was left to stand to allow precipitation for 4 h. After the precipitation completed, the aqueous phase at the lower layer was discharged. Then water was gradually added dropwise (the amount of water added was 15%), and stirring was performed at 200 rpm for 5 min. Then the stirring was stopped, and the mixture was left to stand to allow precipitation for 4 h. After the precipitation was complete, the aqueous phase at the lower layer was discharged.
3. Alkali refining was performed. A sample was taken to determine AV and PV, and the amount of alkali to be added was calculated. The amount of alkali to be added = theoretical alkali (0.713 × oil weight (ton) × acid value / 0.93 alkali concentration) + excess alkali (crude oil weight * 0.0005), and the amount of water to be added (kg) = the amount of alkali to be added (kg) × 40. An alkali solution was prepared using the water and alkali. The oil or fat was heated to 60°C. Stirring was started at 100 rpm, and the alkali solution was added within 5 min. After the addition was completed, the mixture was rapidly stirred for 5 min, and was left to stand to allow precipitation for 4 h to 6 h. After the precipitation was complete, the aqueous phase at the lower layer was discharged. Water washing with salt water: Water at an addition amount of 15% and table salt at an addition amount of 0.5 ‰ were gradually added dropwise to the algae oil. The stirring speed was 100 rpm. After stirring for 3 min, the stirring was stopped, and precipitation was allowed for 3 h. After the precipitation was complete, the aqueous phase at the lower layer was discharged. Water washing with alkali water: Water at an addition amount of 15% and alkali at an addition amount of 0.2 ‰ were gradually added dropwise to the oil or fat. The stirring speed was 100 rpm. After stirring for 3 min, the stirring was stopped, and precipitation was allowed for 3 h. After the precipitation was complete, the aqueous phase at the lower layer was discharged. Water washing with alkali water was repeated twice. Water washing: Water was added at an amount of 15%. After addition, stirring was performed for 3 min. Then, the stirring was stopped, and precipitation was allowed for 3 h. After the precipitation was complete, the aqueous phase at the lower layer was discharged. Water washing was performed twice in total.
4. Decolorization. Decolorization and deodorization were performed according to conventional methods. The temperature for decolorization was 105°C. Carclazyte was added in an amount of about 2% of the oil weight as a decolorization adsorbent. Decolorization was performed at a vacuum degree of 10 mbar for 0.5 h, and then filtering was performed.
5. Deodorization was performed, while introducing nitrogen gas, at a temperature of 200°C and a vacuum degree of 5 mbar for a deodoration time of 2 h. After the refining, refined oil or fat was obtained.

### Oil or Fat 2

OCFA algae oil (Yihai Kerry) and high-oleic sunflower seed oil (Yihai Kerry) were melted and mixed uniformly in a mass ratio of 92:8. Then chemical transesterification was performed such that fatty acids were uniformly distributed on the glycerol backbone, and then the obtained oil or fat was refined. The methods of chemical transesterification and oil refining were the same as those for Oil or Fat 1 (The animal experiments in the present application used the oil or fat obtained by this preparation method).

Oil or Fat 2 could also be prepared according to the following method: Glyceryl tri-undecanoate, glyceryl tri-tridecanoate, glyceryl tri-pentadecanoate and glyceryl tri-heptadecanoate prepared according to the method described above were melted and mixed uniformly with DHA algal oil (Qingdao Keyuan, obtained by refining purchased crude oil, reference was made to "Optimization of the refining processes for microalgae DHA" by Huang Huaixin for the refining method, and the batch of the crude oil was MKD120056) and high-oleic sunflower seed oil (Yihai Kerry) in a mass ratio of 1:3:47:7:35:7. Then chemical transesterification was performed such that fatty acids were uniformly distributed on the glycerol backbone, and then the obtained oil or fat was refined. The methods of chemical transesterification and oil refining were the same as those for Oil or Fat 1.

### Oil or Fat 3:

C15-TAG, Hard ST (Yihai Kerry) and high-oleic sunflower seed oil (Yihai Kerry) were melted and mixed uniformly in a mass ratio of 59:4:37. Then chemical transesterification was performed such that fatty acids were uniformly distributed on the glycerol backbone. The methods of chemical transesterification and oil refining were the same as those for Oil or Fat 1.

The Hard ST and high-oleic sunflower seed oil used in the above Oils or Fats 1 to 3 were products of the same batch from the same manufacturer.

### 4. Fatty acid composition of the oils or fats

The main fatty acid compositions of the above Hard ST, high-oleic sunflower seed oil, DHA algae oil and OCFA algae oil, and the main fatty acid compositions of Oils or Fats 1 to 3, were determined with reference to the third method, i.e., "normalization method" in "GB5009.168-2016 Food Safety National Standard Determination of Fatty Acids in Foods". The results are as shown in Table 1-1 and Table 1-2, respectively.

**Table 1-1: Main fatty acid composition of the Hard ST, high-oleic sunflower seed oil, DHA algae oil and OCFA algae oil**

| Fatty acid | Hard ST | High-oleic sunflower seed oil | DHA algae oil | OCFA algae oil |
|---|---|---|---|---|
| C11:0 | 0.0 | 0.0 | 0.0 | 0.4 |
| C12:0 | 0.1 | 0.2 | 0.1 | 0.1 |
| C13:0 | 0.0 | 0.0 | 0.0 | 3.5 |
| C14:0 | 1.0 | 0.0 | 2.5 | 1.0 |
| C14:1 | 0.0 | 0.0 | 0.3 | 0.4 |
| C15:0 | 0.0 | 0.0 | 0.1 | 51.7 |
| C16:0 | 80.1 | 2.6 | 25.9 | 4.5 |
| C16:1 | 0.0 | 0.0 | 0.2 | 0.4 |
| C17:0 | 0.0 | 0.0 | 0.0 | 7.5 |
| C18:0 | 5.0 | 2.6 | 1.0 | 0.17 |
| C18:1 | 11.7 | 85.8 | 0.6 | 0.5 |
| C18:2 | 1.7 | 8.1 | 0.3 | 0.8 |
| C20:0 | 0.0 | 0.0 | 0.1 | 0.0 |
| C18:3N6 | 0.0 | 0.0 | 0.0 | 0.1 |
| C18:3T | 0.0 | 0.0 | 0.2 | 0.0 |
| C20:1 | 0.0 | 0.0 | 0.0 | 0.0 |
| C18:3 | 0.0 | 0.0 | 0.1 | 0.1 |
| C21:0 | 0.0 | 0.0 | 0.0 | 0.2 |
| C20:2 | 0.0 | 0.0 | 0.3 | 0.2 |
| C22:0 | 0.0 | 0.7 | 0.1 | 0.0 |
| C20:3N6 | 0.0 | 0.0 | 0.1 | 0.1 |
| C22:1 | 0.0 | 0.0 | 0.6 | 0.8 |
| C20:3N3 | 0.5 | 0.0 | 0.2 | 0.1 |
| C20:4N6 | 0.0 | 0.0 | 0.0 | 0.1 |
| C23:0 | 0.0 | 0.0 | 0.6 | 0.5 |
| C22:2 | 0.0 | 0.0 | 0.9 | 0.6 |
| C20:5 | 0.0 | 0.0 | 0.0 | 0.0 |
| C24:0 | 0.0 | 0.0 | 0.0 | 0.2 |
| C24:1 | 0.0 | 0.0 | 0.0 | 0.1 |
| C22:5 | 0.0 | 0.0 | 10.2 | 3.7 |
| C22:6 | 0.0 | 0.0 | 55.3 | 22.3 |

**Table 1-2: Main fatty acid composition of Oils or Fats 1 to 3**

| Fatty acid | Oil or Fat 1 | Oil or Fat 2 | Oil or Fat 3 |
|---|---|---|---|
| **C11:0** | **0.0** | **0.4** | **0.0** |
| C12:0 | 0.1 | 0.1 | 0.0 |
| **C13:0** | **0.0** | **3.2** | **0.0** |
| C14:0 | 0.7 | 0.9 | 0.0 |
| C14:1 | 0.0 | 0.3 | 0.0 |
| **C15:0** | **0.1** | **48.3** | **57.1** |
| C16:0 | 59.4 | 4.5 | 4.8 |
| C16:1 | 0.0 | 0.3 | 0.0 |
| **C17:0** | **0.1** | **7.2** | **0.4** |
| C18:0 | 4.5 | 0.4 | 2.2 |
| C18:1 | 30.7 | 7.8 | 32.2 |
| C18:2 | 3.7 | 0.7 | 3.0 |
| C20:0 | 0.3 | 0.1 | 0.1 |
| C18:3 | 0.1 | 0.2 | 0.2 |
| C20:2 | 0.0 | 0.1 | 0.0 |
| C22:0 | 0.0 | 0.1 | 0.0 |
| C20:3 | 0.3 | 0.1 | 0.0 |
| C22:1 | 0.0 | 0.7 | 0.0 |
| C20:3 | 0.0 | 0.1 | 0.0 |
| C20:4 | 0.0 | 0.0 | 0.0 |
| **C23:0** | **0.0** | **0.4** | **0.0** |
| C22:2 | 0.0 | 0.5 | 0.0 |
| C22:5 | 0.0 | 3.2 | 0.0 |
| C22:6 | 0.0 | 20.3 | 0.0 |
| Total | 100.0 | 100.0 | 100.0 |

### 5. Preparation of oil or fat dietary supplements

15 g of monoglyceride (Yihai Kerry, model DMG-CF01), 15 g of phospholipid (Yihai Kerry, non-transgenic soybean phospholipid) and 300 g of oil or fat (i.e., the above Oil or Fat 1, 2 or 3) were mixed, and were stirred at 60°C until dissolution and uniform mixing. An aqueous solution obtained by dissolving 1.08 kg of lactose and 97.5 g of sodium caseinate in 3.5 kg of pure water was added. High-speed shearing was performed at 12,000 rpm for 8 minutes, and highpressure homogenization was performed at a pressure of 300 bar twice successively, thereby completing the preparation of an emulsion. The above emulsion was spray-dried at an inlet temperature of 180°C and an outlet temperature of 90°C. Oil or fat dietary supplements 1-3 were thus obtained. The oils or fats used in the oil or fat dietary supplements 1 to 3 are shown in Table 2 below.

**Table 2: Oils or fats used in the oil or fat dietary supplements**

| Dietary supplement | Oil or fat used |
|---|---|
| Oil or fat dietary supplement 1 | Oil or Fat 1 |
| Oil or fat dietary supplement 2 | Oil or Fat 2 |
| Oil or fat dietary supplement 3 | Oil or Fat 3 |

### 6. Animal experiments

Experimental animals: In the experiments, healthy "Du × Chang × Da" weaned piglets at the age of 7 days were selected and divided into 3 groups (see Table 3) according to a completely randomized block design based on the principle of similarity in body weight, gender and genetic background, with 10 piglets in each group. A breast-feeding group G0 of homologous piglets raised in a pig farm was additionally set up, with 10 piglets in each group. The 7-day-old piglets were transported from the pig farm to the Institute of Subtropical Agriculture, Chinese Academy of Sciences, fed with commercial piglet formula milk powder (Tang Ren Shen) and allowed to become acclimated to the environment. Daily management was performed according to a management pattern of large-scale pig farms. After 6 days of pre-feeding, the above-mentioned dietary supplements were added every day for the first meal on the basis of the basic formula milk powder (Tang Ren Shen). The piglets were fed to 28 days of age, then fasted for 12 hours, slaughtered and sampled. Samples were taken from 8 piglets in each group for determination and analysis. All related operation procedures met the requirements of the Ethical Committee for Animal Welfare and Animal Experiments of the Institute of Subtropical Agriculture, Chinese Academy of Sciences.

**Table 3: Grouping for experimental treatment**

| Grouping | Ration | Number of animals |
|---|---|---|
| Control group (G0) | Breast milk | 10 |
| Experimental group 1 (G1) | Milk powder + oil or fat dietary supplement 1 | 10 |
| Experimental group 2 (G2) | Milk powder + oil or fat dietary supplement 2 | 10 |
| Experimental group 3 (G3) | Milk powder + oil or fat dietary supplement 3 | 10 |

Methods for determining the nutritional parameters mentioned herein are as follows:
Determination of serum biochemical markers: Commercial kits (Leadman Biotech Limited, Beijing, China) and a biochemical analysis instrument (Beckman CX4; Beckman Coulter, Germany) were used to analyze the concentration of total protein (TP), albumin (ALB), urea nitrogen (BUN), creatinine (CRE), blood glucose (GLU), and insulin (INS) (fasting insulin resistance index = fasting blood glucose × fasting insulin / 22.5, wherein the fasting blood glucose is in units of mmol/L, and the fasting insulin is in units of µU/mL); immunoglobulin IgG, and IgM; HDL-C, LDL-C, serum glutamate aminotransferase (ALT), serum aspartate aminotransferase (AST), serum total triglyceride (TG), total cholesterol (CHOL), D-lactic acid, and total bile acid (TBA).

Indexes for RT-PCR determination: IL-1β, IL-6, TNF-α, TLR4, NF-κB, and MUC-2 in the duodenum, jejunum, and ileum. SREBP-1C, PPARγ, APOB, FAS, ACC, PPARα, ACOX, CPT-1α, HSL, etc. in the longissimus dorsi muscle and dorsal fat. For the method of RT-QPCR, reference was made to "B. Song, Dietary leucine supplementation improves intestinal health of mice through intestinal SIgA secretion, 2019".

Indexes for western blot analysis: Ki-67, Occludin, Claudin, ZO-1, TLR4, NK-κB, mTOR, ULK1, TFEB, SREBP1, PPARγ, HIF1α, PINK, Parkin, and p-mTOR in each intestinal segment; p-mTOR, Prdm16, and Irisin in dorsal fat and perirenal fat; Slow MyHC, Fast MyHC, mTOR, AMPK, PGC-1α in dorsal muscles. For the method of western blot analysis, reference was made to "B. Song, Dietary leucine supplementation improves intestinal health of mice through intestinal SIgA secretion, 2019".

Indexes for ELISA assay: Ground tissue was determined according to the manufacturer's instructions (Nanjing Jiancheng Bioengineering Institute, Jiangsu, China). Commercial porcine ELISA kits (Cusabio Life Science, Inc., Wuhan, China) were used to determine serum malondialdehyde MDA, glutathione peroxidase GSH-PX, superoxide dismutase SOD; insulin and glucagon; the concentration of stress hormones (cortisol and ACTH), D-xylose, IL-1β, IL-6, IL-10, and TNF-α. IL-1β, IL-6, IL-10, and TNF-α in each intestinal segment. IgA, IgG, and IgM in the spleen, jejunum, and ileum. The activity of aminopeptidase N, sucrase and maltase in each intestinal segment, the expression of 5-hydroxytryptamine, noradrenaline, Substance P, corticotropic hormone-releasing factor. IgE and HIS in the plasma, spleen and each intestinal segment.

Morphology of the intestinal tract: Reference was made to "J. Y. Yu, Development of intestinal injury and restoration of weaned piglets under chronic immune stress, 2022" and "J. Wang, Developmental changes in intercellular junctions and Kv channels in the intestine of piglets during the suckling and post-weaning periods, 2016". Hematoxylin-eosin staining was used to perform morphological analysis on fixed segments of the duodenum, jejunum, and ileum. Parameters such as villus height (VH) and crypt depth (CD) were determined under a microscope and measured by Case Viewer software. The ratio of VH to CD was calculated. Reference was made to "Y. Xiao, Ellagic acid alleviatesoxidative stress by mediating Nrf2 signaling pathways and protects against paraquat-induced intestinal injury in piglets". An intestinal segment was cold fixed with 2.5% glutaraldehyde to perform scanning and transmission electron microscopy analysis.

### 7. Results

### 7.1 Health of the intestinal tract

### (1) Mechanical barrier of the intestinal tract

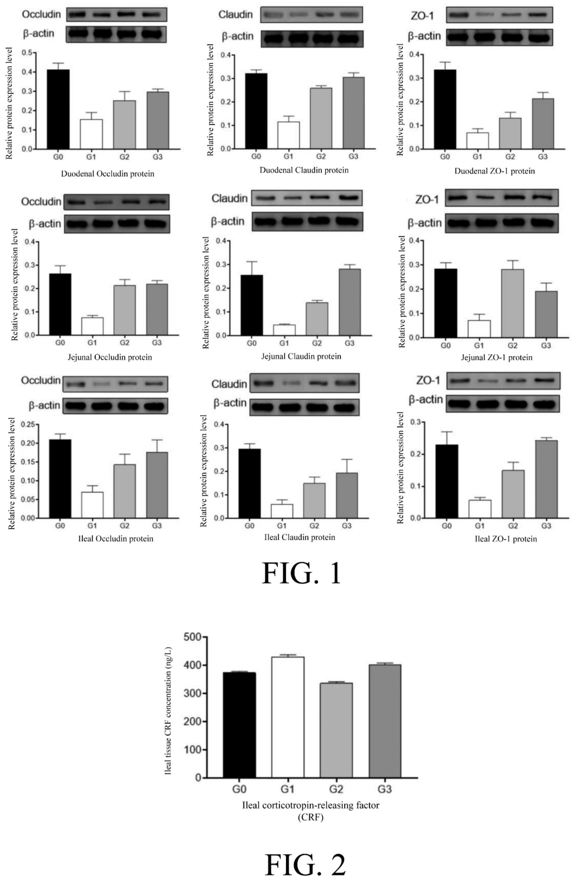

Table 4 and Fig. 1 show the effects of daily ration treatment on the expression of tight-junction proteins in the intestinal tracts of piglets. As can be seen from Fig. 1, compared with the breast milk group (G0 group), the G1 group had significantly reduced protein expression abundance of tight junction-associated proteins (Occludin, Claudin and ZO-1) in the duodenal, jejunal and ileal mucosa (P < 0.05). Compared with the G1 group, the G3 group had significantly increased protein expression abundance of tight junction-associated proteins (Occludin, Claudin and ZO-1) in the duodenal, jejunal and ileal mucosa (P < 0.05). Compared with the G1 group, the protein expression abundance of tight junction-associated proteins (Occludin, Claudin and ZO-1) in the duodenal, jejunal and ileal mucosa of the G2 group was also increased. These results indicate that, compared with even-carbon fatty acids, diet supplementation with odd-carbon fatty acids can improve the mechanical barrier of the intestinal tract of piglets by promoting the expression of tight junction proteins in intestinal epithelial cells; in the composition of the odd-carbon chain fatty acids, the higher the proportion of C15, the more the mechanical barrier of the intestinal tract of piglets can be improved by promoting the expression of tight junction proteins (such as duodenal Occludin, duodenal Claudin, duodenal ZO-1, jejunal Occludin, jejunal Claudin, ileal Occludin, ileal Claudin, and ileal ZO-1) in intestinal epithelial cells.

**Table 4**

| | G0 | G1 | G2 | G3 |
|---|---|---|---|---|
| Duodenal Occludin protein | 0.41 | 0.16 | 0.25 | 0.30 |
| Duodenal Claudin protein | 0.32 | 0.12 | 0.26 | 0.31 |
| Duodenal ZO-1 protein | 0.34 | 0.07 | 0.13 | 0.21 |
| Jejunal Occludin protein | 0.26 | 0.08 | 0.21 | 0.22 |
| Jejunal Claudin protein | 0.26 | 0.05 | 0.14 | 0.28 |
| Jejunal ZO-1 protein | 0.28 | 0.07 | 0.28 | 0.19 |
| Ileal Occludin protein | 0.21 | 0.07 | 0.14 | 0.18 |
| Ileal Claudin protein | 0.30 | 0.06 | 0.15 | 0.19 |
| Ileal ZO-1 protein | 0.23 | 0.06 | 0.15 | 0.24 |

Table 5 and Fig. 2 show the effects of daily ration treatment on the levels of a releasing hormone in the intestinal tract of piglets. Corticotropin-releasing hormone (CRF) is a releasing hormone involved in stress response, and is mainly used to promote the synthesis of corticotropin in the pituitary gland. It is often believed that CRF is only related to central system secretion, but the evidence of the present application indicates that peripheral release of CRF plays a crucial role in the regulation of the permeability of the gastrointestinal tract, and is part of the connections of the gut-brain axis. As can be seen from Fig. 2, compared with the breast milk group (G0 group), the G1 group had significantly increased CRF content in the ileum of the piglets (P < 0.05), suggesting that the mechanical barrier of the intestinal wall at the end of the small intestine might have been weakened. However, compared with the G1 group, the CRF content in the ileal tissue of the piglets in the G3 group was markedly reduced (P < 0.05), and the CRF content in the ileal tissue of the piglets in the G2 group was also reduced compared with the G1 group. These results indicate that, compared with even-carbon fatty acids, diet supplementation with odd-carbon fatty acids can also enhance or improve the mechanical barrier function of the intestinal tract of piglets by reducing the content of CRF in a tissue of the intestinal tract.

**Table 5**

| | G0 | G1 | G2 | G3 |
|---|---|---|---|---|
| Ileal corticotropin-releasing factor (CRF) | 374.17 | 430.98 | 336.85 | 403.84 |

### (2) Immune homeostasis in the intestinal tract

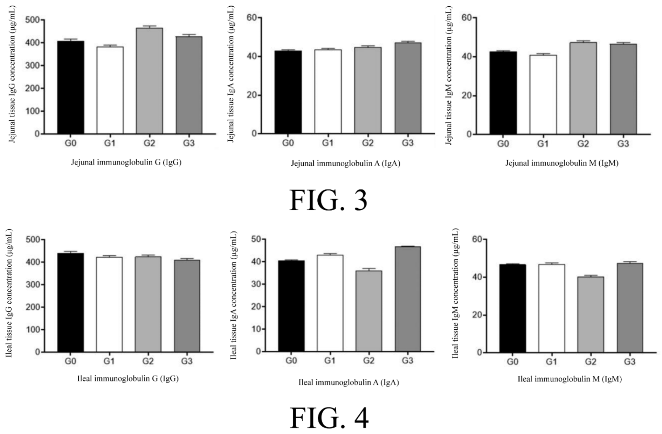

Table 6-1 and Fig. 3 show the effects of daily ration treatment on the jejunal immunoglobulins of piglets. Immunoglobulins are a class of proteins having antibody activity in the human body. Immunoglobulins are an important component of the body's resistance to disease. They have antibacterial and antiviral effects and enhance the phagocytosis of cells, and, with the synergy of complements, can kill or dissolve pathogenic microorganisms. Typically, IgG, IgM and IgA are detected. IgG, which accounts for 75% of total immunoglobulins, is the most long-lasting and important antibody in the primary immune response. IgA is the first line of defense against pathogen invasion. IgM plays an important role in early defense. As can be seen from Fig. 3, compared with the G1 group, IgG, IgA, and IgM in the jejunum of the G3 group were all significantly increased (P < 0.05); compared with the G1 group, jejunal IgG protein and IgM protein were also increased in the G2 group. These results suggest that, compared with even-carbon fatty acids, dietary supplementation with odd-carbon fatty acids can maintain the immune homeostasis of the jejunum by increasing the amount of jejunal immunoglobulins.

**Table 6-1**

| | G0 | G1 | G2 | G3 |
|---|---|---|---|---|
| Jejunal immunoglobulin G (IgG) | 409.07 | 383.43 | 465.45 | 426.07 |
| Jejunal immunoglobulin A (IgA) | 42.96 | 43.61 | 44.71 | 46.90 |
| Jejunal immunoglobulin M (IgM) | 42.69 | 40.79 | 47.35 | 46.09 |

Table 6-2 and Fig. 4 show the effects of daily ration treatment on the ileal immunoglobulins of piglets. As can be seen from Fig. 4, compared with the G1 group, the G3 group had significantly increased IgA in the ileum (P < 0.05), and there was no significant change in IgG and IgM. IgA is the first line of defense against pathogen invasion, indicating that, compared with even-carbon fatty acids, dietary supplementation with odd-carbon fatty acids can enhance the immunity of the jejunum by increasing the content of immunoglobulin IgA in the ileum, to improve the ability of the ileum to defend against pathogen invasion.

**Table 6-2**

| | G0 | G1 | G2 | G3 |
|---|---|---|---|---|
| Ileal immunoglobulin G (IgG) | 440.25 | 422.98 | 424.97 | 409.30 |
| Ileal immunoglobulin A (IgA) | 40.63 | 43.11 | 36.09 | 46.13 |
| Ileal immunoglobulin M (IgM) | 46.72 | 46.93 | 40.37 | 47.56 |

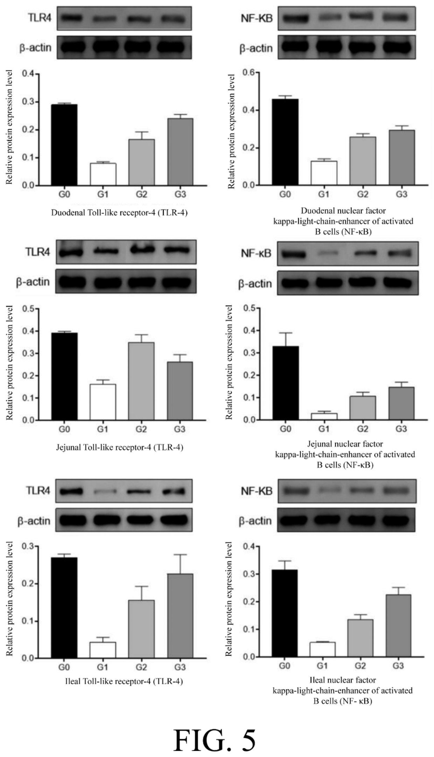

Table 7 and Fig. 5 show the effects of daily ration treatment on the TLR4/NF-κB signaling pathway in the intestinal tracts of piglets. Inflammatory responses are an important immune defense mechanism of the body. Normal inflammatory responses are a manifestation of the body's immunity, and are beneficial to the body. TLR4/NF-κB is an inflammatory response signaling pathway of the body. As can be seen from Fig. 5, compared with the breast milk group (G0 group), the protein expression abundance of inflammatory response-associated genes (TLR4 and NF-κB) in the duodenum, jejunum, and ileum of the piglets in the G1 group was significantly reduced (P < 0.05), indicating that the change in the diet led to a change in the immune homeostasis in the intestinal tract. Compared with the G1 group, the G3 group had significantly up-regulated protein expression abundance of TLR4 and NF-κB in the duodenum, jejunum, and ileum (P < 0.05); compared with the G1 group, the protein expression abundance of TLR4 and NF-κB in the duodenum, jejunum, and ileum of the G2 group was also up-regulated. These results suggest that compared with even-carbon fatty acids, dietary supplementation with odd-carbon fatty acids can maintain the immune homeostasis in the intestinal tract by regulating the expression of the TLR4/NK-κB signaling pathway; in the composition of the odd-carbon chain fatty acids, the higher the proportion of C15, the more the immune homeostasis in the intestinal tract can be maintained by regulating the expression of the TLR4/NK-κB signaling pathway.

**Table 7**

| | G0 | G1 | G2 | G3 |
|---|---|---|---|---|
| Duodenal Toll-like receptor-4 (TLR-4) | 0.29 | 0.08 | 0.17 | 0.24 |
| Duodenal nuclear factor kappa-light-chain-enhancer of activated B cells (NF-kB) | 0.46 | 0.13 | 0.26 | 0.29 |
| Jejunal Toll-like receptor-4 (TLR-4) | 0.39 | 0.16 | 0.35 | 0.26 |
| Jejunal nuclear factor kappa-light-chain-enhancer of activated B cells (NF-kB) | 0.33 | 0.03 | 0.11 | 0.15 |
| Ileal Toll-like receptor-4 (TLR-4) | 0.27 | 0.04 | 0.16 | 0.23 |
| Ileal nuclear factor kappa-light-chain-enhancer of activated B cells (NF-kB) | 0.32 | 0.05 | 0.14 | 0.23 |

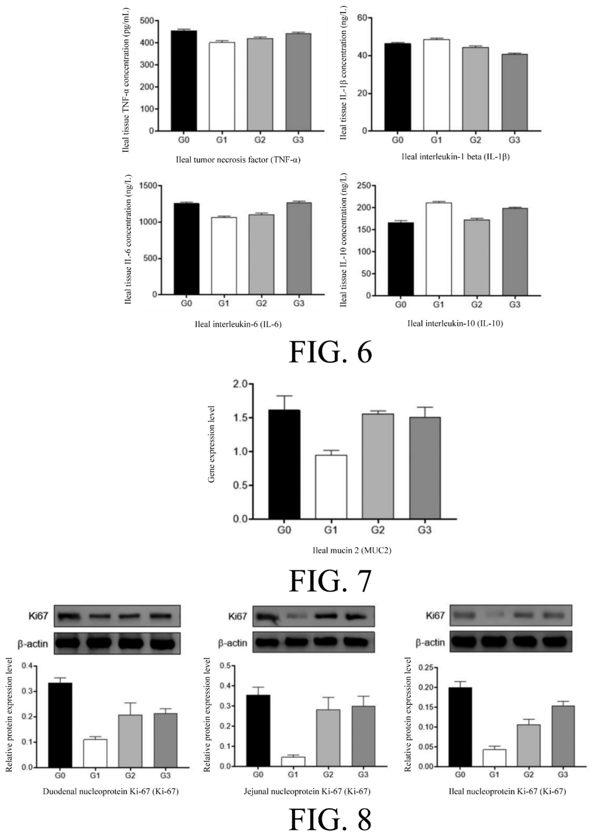

Table 8 and Fig. 6 show the effects of daily ration treatment on the ileal inflammatory cytokines (TNF-α, IL-1β, IL-6 and IL-10) of piglets. Inflammation induces damaged cells to release chemical substances. These released chemical substances are commonly referred to as inflammatory cytokines. Cytokines, as information molecules between cells, interact with cells of the immune system, regulate the body's responses to disease and infection, and mediate the activity of the body's normal cells. As can be seen from Fig. 6, compared with the breast milk group (G0 group), the ileal inflammatory cytokines in the G1 group were changed (P < 0.05), indicating that the change in the diet led to a change in the immune homeostasis of the ileum. Compared with G1, the G3 and G2 groups could modulate the inflammatory cytokines to the extent that the inflammatory cytokines were close to those of the breast milk group. This indicates that, compared with even-carbon fatty acids, dietary supplementation with odd-carbon fatty acids can maintain the immune homeostasis of the ileum by modulating the inflammatory cytokines.

**Table 8**

| | G0 | G1 | G2 | G3 |
|---|---|---|---|---|
| Ileal tumor necrosis factor (TNF-a) | 456.00 | 401.73 | 420.40 | 441.79 |
| Ileal interleukin-1 beta (IL-1β) | 46.41 | 48.58 | 44.42 | 40.74 |
| Ileal interleukin-6 (IL-6) | 1258.04 | 1065.78 | 1104.21 | 1266.52 |
| Ileal interleukin-10 (IL-10) | 166.54 | 211.31 | 173.33 | 198.35 |

Table 9 and Fig. 7 show the effect of daily ration treatment on the level of ileal mucin 2 in piglets. Mucin 2 (MUC-2) is one of the main components of the mucus layer of the intestinal tract, and is secreted by goblet cells. It can form a colloidal matrix containing antibacterial molecules, thereby effectively preventing the invasion of bacteria, viruses, etc. into the intestinal mucosa. As shown in Fig. 7, compared with the breast milk group (G0 group), the expression amount of ileal mucin 2 in the G1 group was significantly decreased, suggesting that the immune function of the ileal mucosa was reduced (P < 0.05). However, the G2 group and the G3 group modulated the expression amount of ileal mucin 2 to the extent that the expression amount restored to the level of the breast milk group. This indicates that, compared with even-carbon fatty acids, dietary supplementation with odd-carbon fatty acids can also jointly maintain the immune homeostasis of the ileum by maintaining the level of mucin in the mucosa.

**Table 9**

| | G0 | G1 | G2 | G3 |
|---|---|---|---|---|
| Ileal mucin 2 (MUC2) | 1.62 | 0.95 | 1.57 | 1.52 |

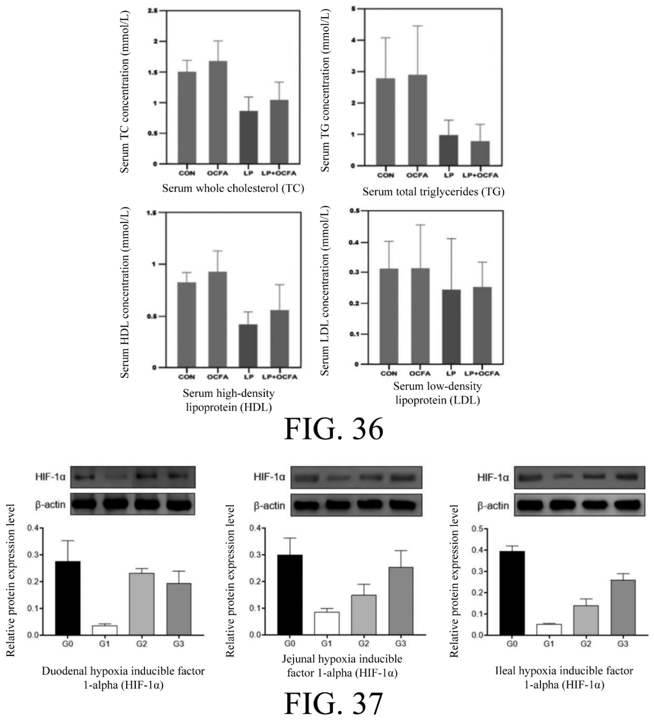

Table 10 and Fig. 37 show the effects of daily ration treatment on the relative expression of hypoxia-inducible factor HIF-1α in the intestinal tracts of piglets. The environment of the intestinal tract is usually in a hypoxic state. HIF-1α, as a major regulatory factor of cellular responses to hypoxia, plays an important role in the homeostasis and host-microorganism interaction in the intestinal tract. For example, some of its target genes may be involved in the barrier function of the intestinal tract. As can be seen from Fig. 37, the expression of HIF-1α in the G3 group was significantly higher than in the G1 group. This indicates that, compared with even-carbon fatty acids, dietary supplementation with odd-carbon fatty acids can increase the expression of HIF-1α in the intestinal tract, thereby promoting the survival of cells of the intestinal tract in the hypoxic environment of the intestinal tract, and benefiting the homeostasis of the intestinal tract.

**Table 10**

| | G0 | G1 | G2 | G3 |
|---|---|---|---|---|
| Duodenal hypoxia-inducible factor 1-alpha (HIF-1α) | 0.28 | 0.04 | 0.23 | 0.19 |
| Jejunal hypoxia-inducible factor 1-alpha (HIF-1α) | 0.30 | 0.09 | 0.15 | 0.25 |
| Ileal hypoxia-inducible factor 1-alpha (HIF-1α) | 0.40 | 0.05 | 0.14 | 0.26 |

### (3) Development of the intestinal tract

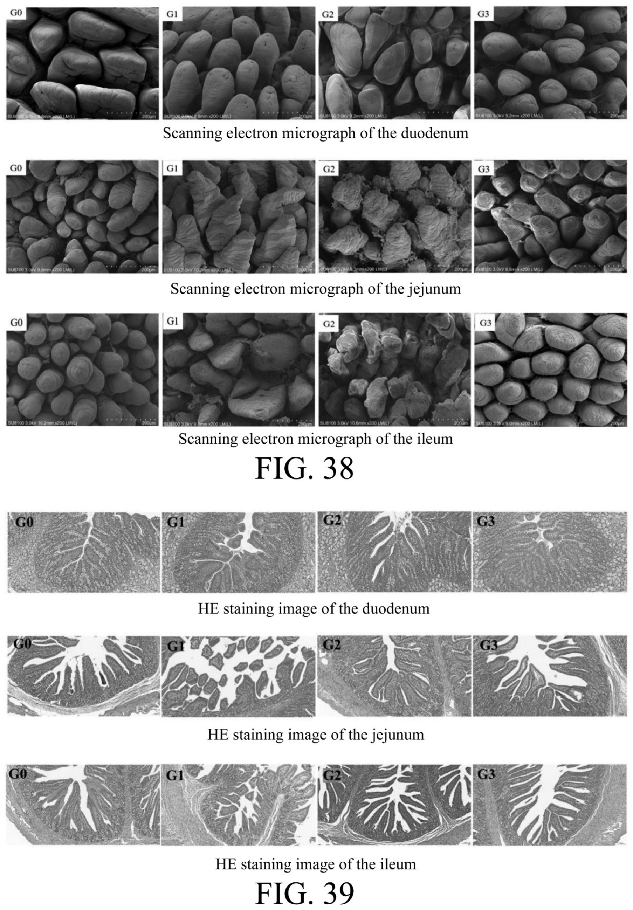

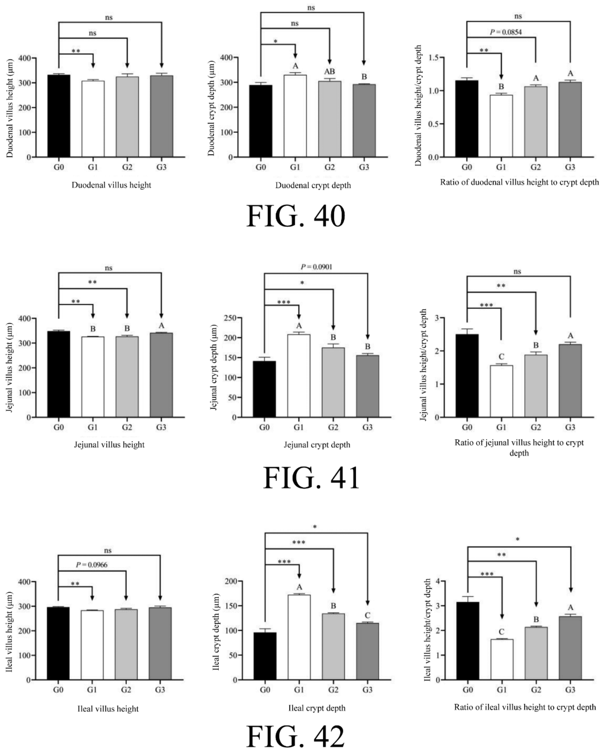

Figs. 38-42 show the determination results related to the development of the intestinal tract. Scanning electron microscopy (Fig. 38) shows that the intestinal villi in the piglets of the G2 and G3 groups were longer, thicker and denser than those in the G1 group. Especially, the intestinal villi in the G3 group was comparable to that in the G0 group. The morphology of the intestinal tract under HE staining is shown in Fig. 39. The duodenum, jejunum, and ileum of the piglets in the G2 group and the G3 group all showed a morphology of the intestinal tract better than that in the G1 group and closer to that of the piglets in the breast-fed G0 group. The functional units of the small intestine are villi. Longer villi have more absorptive cells, and deeper crypts result in less contact between enterocytes and nutrients. The villus height (VH) and the ratio of the villus height to the crypt depth (CD) (VH:CD) of the small intestine are often used as indexes for evaluating the development condition of the small intestine. As can be seen from the results in Figs. 40-42, the VH and VH:CD of the intestinal tract of the piglets in the G1 group were both significantly lower than those in the G0 group (both P < 0.05), and the CD was deeper (both P < 0.05), indicating that feeding with milk powder can adversely affect the intestinal tract of piglets. As shown in Fig. 40 and Table 11-1, in the duodenum, the VH:CD of the G2 and G3 groups were significantly higher than that of the G1 group (P < 0.05), and none of the VH, CD and VH:CD of the G2 and G3 groups was significantly different from those of the G0 group. As shown in Fig. 41 and Table 11-2, in the jejunum, the VH:CD of the G2 group was significantly higher than that of the G1 group (P < 0.05), and the CD was significantly lower than that of the G1 group (P < 0.05). The VH and VH:CD of the G3 group were significantly higher than those of the G1 group (P < 0.05), and the CD was significantly lower than that of the G1 group (P < 0.05). Moreover, none of the foregoing indexes was significantly different from those of the G0 group. As shown in Fig. 42 and Table 11-3, in the ileum, the VH:CD of the G2 and G3 groups was significantly higher than that of the G1 group (P < 0.05), and the CD was significantly lower than that of the G1 group (P < 0.05). Moreover, the foregoing indexes were close to those of the G0 group. In summary, the above results demonstrate that odd-carbon fatty acids are beneficial to improving the morphology of the intestinal tract, increasing VH and VH:CD, and decreasing CD, and are beneficial to the development of the intestinal tract, enabling the development of the intestinal tract of the animals fed with milk powder to reach a level equivalent to that of the breast milk group.

**Table 11-1**

| | G0 | G1 | G2 | G3 |
|---|---|---|---|---|
| Duodenal villus height | 332.25 | 308.14 | 324.97 | 329.91 |
| Duodenal crypt depth | 289.28 | 330.00 | 305.28 | 292.43 |
| Ratio of duodenal villus height to crypt depth | 1.15 | 0.94 | 1.06 | 1.13 |

**Table 11-2**

| | G0 | G1 | G2 | G3 |
|---|---|---|---|---|
| Jejunal villus height | 348.35 | 326.15 | 327.21 | 341.69 |
| Jejunal crypt depth | 141.41 | 208.38 | 175.09 | 155.70 |
| Ratio of jejunal villus height to crypt depth | 2.50 | 1.57 | 1.89 | 2.20 |

**Table 11-3**

| | G0 | G1 | G2 | G3 |
|---|---|---|---|---|
| Ileal villus height | 296.40 | 283.48 | 287.37 | 295.29 |
| Ileal crypt depth | 96.12 | 172.30 | 134.43 | 115.12 |
| Ratio of Ileal villus height to crypt depth | 3.15 | 1.65 | 2.14 | 2.57 |

Table 12 and Fig. 8 show the effect of daily ration treatment on the proliferation of the epithelial cells of the intestinal tracts of piglets. Ki67 is an antigen related to proliferating cells. Its function is closely related to mitosis and is indispensable in cell proliferation. A higher expression level of Ki67 represents more active cell proliferation. As can be seen from Fig. 8, the protein expression abundance of Ki67 in the intestinal epithelial cells of the G3 group was significantly higher than that of the G1 group (P < 0.05), and the protein expression abundance of Ki67 in the intestinal epithelial cells of the G2 group was significantly higher than that of the G1 group. These results indicate that, compared with even-carbon fatty acids, diet supplementation with odd-carbon fatty acids can improve the mechanical barrier of the intestinal tract and promote the development of the intestinal tract by promoting the proliferation of the intestinal epithelial cells; in the composition of the odd-carbon chain fatty acids, the higher the proportion of C15, the more the mechanical barrier of the intestinal tract can be improved, and the development of the intestinal tract can be promoted by promoting the proliferation of the intestinal epithelial cells.

**Table 12**

| | G0 | G1 | G2 | G3 |
|---|---|---|---|---|
| Duodenal nucleoprotein Ki-67 (Ki-67) | 0.33 | 0.11 | 0.21 | 0.21 |
| Jejunal nucleoprotein Ki-67 (Ki-67) | 0.36 | 0.05 | 0.28 | 0.30 |
| Ileal nucleoprotein Ki-67 (Ki-67) | 0.20 | 0.04 | 0.11 | 0.15 |

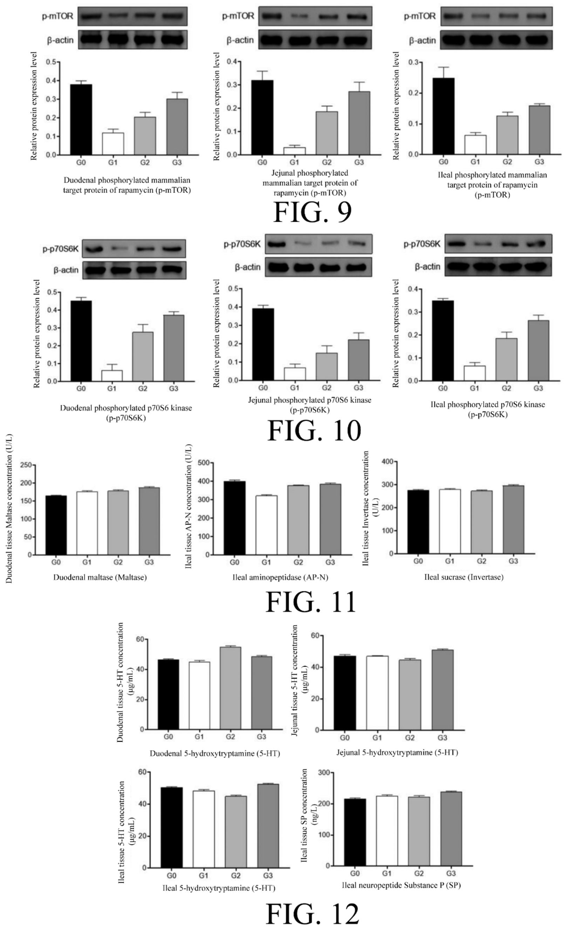

Table 13 and Fig. 9 show the effect of daily ration treatment on the mTOR protein level in the small intestines of piglets. mTOR (mammalian target of rapamycin) protein is a kinase, which is regulated by growth factors and environmental nutrient levels. It can interact with various proteins, activate or inhibit downstream signaling pathways, thereby promoting the production of ribosomes and the synthesis of proteins, lipids and nucleic acids, inhibiting cellular autophagy, promoting the growth and proliferation of cells, among others, thus playing an important role in regulating cellular processes and tissue development. Compared with the breast milk group (G0 group), the expression amount of phosphorylated (activated) mTOR protein in the G1 group was significantly decreased (P < 0.05), indicating that the normal growth activity of cells was inhibited. The G3 group could increase the expression amount of activated mTOR protein in cells of the small intestine to the extent that the expression amount reached or was close to that of the breast milk group; in addition, the expression amount of activated mTOR protein in cells of the small intestine of the G2 group was also higher than that of the G1 group. These results indicate that dietary supplementation with odd-carbon fatty acids can activate the mTOR signaling pathway, aiding in cell growth and tissue development; in the composition of the odd-carbon chain fatty acids, the higher the proportion of C15, the more the mTOR signaling pathway can be activated, which aids in cell growth and tissue development.

**Table 13**

| | G0 | G1 | G2 | G3 |
|---|---|---|---|---|
| Duodenal phosphorylated mammalian target protein of rapamycin (p-mTOR) | 0.38 | 0.12 | 0.21 | 0.30 |
| Jejunal phosphorylated mammalian target protein of rapamycin (p-mTOR) | 0.32 | 0.03 | 0.19 | 0.27 |
| Ileal phosphorylated mammalian target protein of rapamycin (p-mTOR) | 0.25 | 0.06 | 0.13 | 0.16 |

Table 14 and Fig. 10 show the effect of treatment on the level of p70S6K in the small intestines of piglets. p70S6K (p70 ribosomal S6 kinases) is a class of protein kinases involved in signal transduction, and can be activated by mTOR protein to regulate cell proliferation and tissue development by regulating protein synthesis and ribosome biogenesis. Similar to the state of the expression of mTOR protein, the level of phosphorylated (activated) S6 kinases in the G1 group was significantly decreased (P < 0.05) compared with the breast milk group (G0 group), indicating that the normal growth of cells was inhibited. The G3 group could increase the level of activated S6 kinases in cells of the small intestine to the extent that the level reached or was close to that of the breast milk group, and the level of activated S6 kinases in cells of the small intestine of the G2 group was also markedly higher than that of the G1 group. This indicates that dietary supplementation with odd-carbon fatty acids can save cell proliferation and protein metabolism in tissue of the small intestine and promote tissue development. This regulation might be mediated by the mTOR signaling pathway. In the composition of the odd-carbon chain fatty acids, the higher the proportion of C15, the more the cell proliferation and protein metabolism in a tissue of the small intestine can be saved, and tissue development can be promoted.

**Table 14**

| | G0 | G1 | G2 | G3 |
|---|---|---|---|---|
| Duodenal phosphorylated p70S6 kinase (p-p70S6K) | 0.45 | 0.06 | 0.28 | 0.37 |
| Jejunal phosphorylated p70S6 kinase (p-p70S6K) | 0.39 | 0.07 | 0.15 | 0.22 |
| Ileal phosphorylated p70S6 kinase (p-p70S6K) | 0.35 | 0.07 | 0.19 | 0.26 |

Table 15 and Fig. 11 show the effect of daily ration treatment on the level of digestive enzymes in the small intestines of piglets. Maltase, aminopeptidase (APN) and sucrase (Invertase) are digestive enzymes on the brush border of the small intestine. They can reflect the digestive and absorptive functions of the intestinal tract and are important markers of the physiological functions of the intestinal tract and the development and maturation thereof. As can be seen from Fig. 11, compared with the breast milk group (G0 group), the levels of the digestive enzymes in the G1 group were significantly different (P < 0.05), indicating that the change in the diet led to a change in the digestive capacity of the small intestine. Group G3 could increase the levels of the digestive enzymes in the small intestine to the extent that the levels reached or even exceeded those in the breast milk group. This indicates that dietary supplementation with odd-carbon fatty acids can aid in the sufficient absorption of nutrients by increasing the levels of digestive enzymes in the small intestine.

**Table 15**

| | G0 | G1 | G2 | G3 |
|---|---|---|---|---|
| Duodenal maltase (Maltase) | 164.88 | 176.14 | 178.74 | 187.66 |
| Ileal aminopeptidase N (AP-N) | 400.69 | 322.05 | 384.06 | 384.75 |
| Ileal sucrase (Invertase) | 275.93 | 279.51 | 273.79 | 296.57 |

Table 16 and Fig. 12 show the effect of daily ration treatment on the levels of a neurotransmitter and a neuropeptide in the small intestines of piglets. 5-hydroxytryptamine (5-HT) is a kind of monoamine neurotransmitter. It is mainly synthesized in the chromaffin cells of the small intestine of animals, and is also present in small amounts in platelets and the central nervous system, playing different functions in different tissues. About 90% of the 5-hydroxytryptamine in the animal body is present in gastrointestinal cells and the myenteric nerve plexus, and is involved in the regulation of intestinal peristalsis. Substance P (SP) is also widely distributed in the gastrointestinal tract, and mainly acts to promote the contraction of the gastrointestinal smooth muscle, inhibit the secretion of gastric acid and bile, and regulate gastrointestinal local blood flow, etc. As shown in the foregoing figure, the contents of the neurotransmitter and the neuropeptide in the small intestine of the G1 group were similar or slightly decreased compared with those of the breast milk group (G0 group). However, the G3 group could significantly increase the levels of 5-hydroxytryptamine in the duodenum, jejunum, and ileum and the amount of Substance P in the ileum to the extent that they were restored to the levels of the breast milk group or above, and the G2 group could significantly increase the level of 5-hydroxytryptamine in the duodenum. This suggests that dietary supplementation with odd-carbon fatty acids can enhance the functions of the small intestine by increasing the levels of gut-brain peptides related to the gut-brain axis.

**Table 16**

| | G0 | G1 | G2 | G3 |
|---|---|---|---|---|
| Duodenal 5-hydroxytryptamine (5-HT) | 46.45 | 45.44 | 55.01 | 48.54 |
| Jejunal 5-hydroxytryptamine (5-HT) | 47.26 | 47.74 | 44.63 | 50.67 |
| Ileal 5-hydroxytryptamine (5-HT) | 50.57 | 48.28 | 44.90 | 52.44 |
| Ileal neuropeptide Substance P (SP) | 216.94 | 225.28 | 222.05 | 238.48 |

### (4) Energy metabolism of the intestinal tract

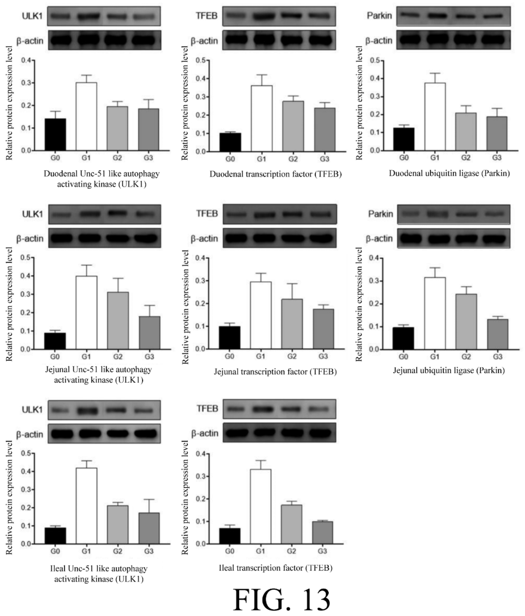

Table 17 and Fig. 13 show the effect of daily ration treatment on the levels of mitochondrial autophagy in the small intestines of piglets. Mitochondrial autophagy is a process of intracellular programmed degradation of mitochondria, which mostly occurs in damaged or defective mitochondria, and is used to prevent cell abnormalities resulting from the accumulation of dysfunctional mitochondria. As shown in Fig. 13, compared with the breast milk group (G0 group), the expression amounts of key proteins of mitochondrial autophagy (ULK1, TFEB and Parkin) in the G1 group were all significantly increased (P < 0.05), suggesting excessive accumulation of abnormal mitochondria in cells. The G2 and G3 groups could down-regulate the expression amounts of intracellular mitochondrial autophagy-associated proteins to the extent of reaching a level similar to that of the breast milk group. This indicates that compared with even-carbon fatty acids, dietary supplementation with odd-carbon fatty acids can alleviate mitochondrial dysfunction, which would be beneficial to maintaining the energy homeostasis of cells of the intestinal tract; in the composition of the odd-carbon chain fatty acids, the higher the proportion of C15, the more the mitochondrial dysfunction can be alleviated, which would be beneficial to maintaining the energy homeostasis of cells of the intestinal tract.

**Table 17**

| | G0 | G1 | G2 | G3 |
|---|---|---|---|---|
| Duodenal Unc-51 like autophagy activating kinase (ULK1) | 0.14 | 0.30 | 0.20 | 0.19 |
| Duodenal transcription factor EB (TFEB) | 0.10 | 0.36 | 0.28 | 0.24 |
| Duodenal ubiquitin ligase (Parkin) | 0.13 | 0.38 | 0.21 | 0.19 |
| Jejunal Unc-51 like autophagy activating kinase (ULK1) | 0.09 | 0.40 | 0.31 | 0.18 |
| Jejunal transcription factor EB (TFEB) | 0.10 | 0.30 | 0.22 | 0.18 |
| Jejunal ubiquitin ligase (Parkin) | 0.10 | 0.32 | 0.24 | 0.13 |
| Ileal Unc-51 like autophagy activating kinase (ULK1) | 0.09 | 0.42 | 0.21 | 0.17 |
| Ileal transcription factor EB (TFEB) | 0.07 | 0.33 | 0.17 | 0.10 |

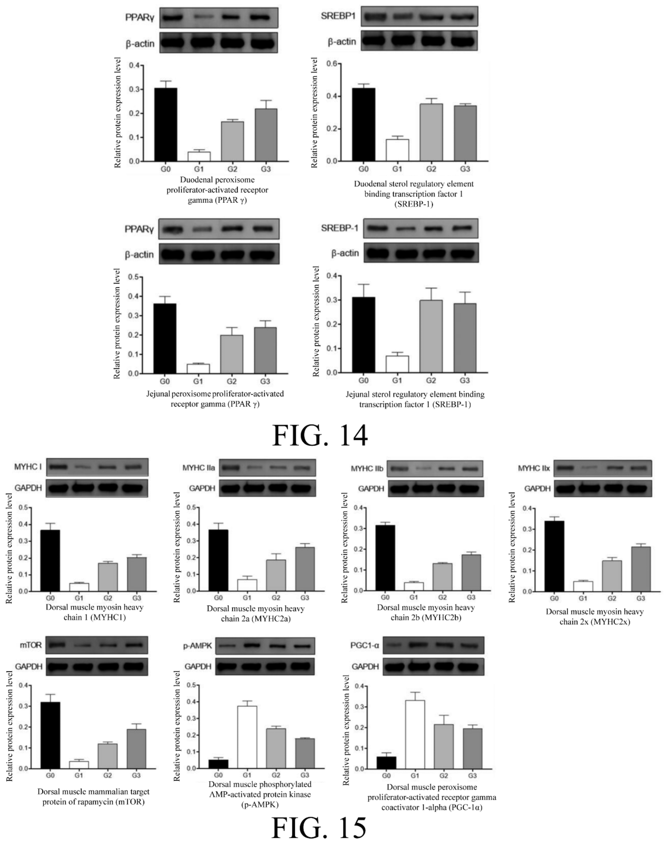

Table 18 and Fig. 14 show the effect of daily ration treatment on the levels of lipid metabolism in the intestinal tracts of piglets. Lipids are important functional substances in the body and cells, and are a main form of energy storage. The degree of activity of lipid metabolism can reflect the energy state of an entire tissue. PPARγ and SREBP-1 are transcription factors involved in lipid metabolism in cells, and are closely related to lipid uptake and synthesis. As can be seen from the foregoing figure, compared with the breast milk group (G0 group), the contents of PPARγ and SREBP-1 in the duodenum and jejunum of the G1 group were significantly reduced (P < 0.05), suggesting that lipid synthesis was inhibited and there was an abnormality in the cellular energy state. The G2 group and G3 group could up-regulate the levels of the transcription factors related to lipid synthesis, and to some extent, the levels reached or approached the levels of the breast milk group. This indicates that dietary supplementation with odd-carbon fatty acids can increase the absorption and anabolism of lipids in the small intestine of piglets, which would be beneficial to maintaining the energy homeostasis of cells of the intestinal tract; in the composition of the odd-carbon chain fatty acids, the higher the proportion of C15, the more the absorption and anabolism of lipids in the small intestine of piglets can be increased, which would be beneficial to maintaining the energy homeostasis of cells of the intestinal tract.

**Table 18**

| | G0 | G1 | G2 | G3 |
|---|---|---|---|---|
| Duodenal peroxisome proliferator-activated receptor gamma (PPARγ) | 0.31 | 0.04 | 0.17 | 0.22 |
| Duodenal sterol regulatory element binding transcription factor 1 (SREBP-1) | 0.45 | 0.14 | 0.35 | 0.34 |
| Jejunal peroxisome proliferator-activated receptor gamma (PPARγ) | 0.36 | 0.05 | 0.20 | 0.24 |
| Jejunal sterol regulatory element binding transcription factor 1 (SREBP-1) | 0.31 | 0.07 | 0.30 | 0.29 |

### (5) Development and metabolism of muscle tissue

Muscle tissue is composed of muscle fibers (cells), and muscle cells can be classified into type I fibers (MYHC I) and type II fibers (MYHC II) according to the differences in their appearance and function. Type I fibers are also called red muscle fibers, slow muscle fibers or slow oxidation fibers. Such muscle fibers have a slow contraction speed, a small force, good oxygen utilization, and good endurance. Type II fibers are called white muscle fibers, fast muscle fibers or fast glycolytic fibers. They mainly provide short-term energy through glycolysis and have poor endurance, but they have a strong effect on improving the explosive power and speed of exercise, and are also a main factor in determining muscle strength and speed. In addition, the muscle cross-section of the fast muscle fibers is relatively thick, and muscle tissue rich in fast muscle fibers tends to be well-developed and thick. In animal nutrition, the number of fast muscle fibers is significantly related to the meat yield of animals, and is also a closely watched indicator in animal production. mTOR also plays an important function in muscle tissue, and can control the anabolic and catabolic signals of skeletal muscle, thereby achieving the regulation of muscle enlargement and muscle atrophy. AMPK (AMP-activated protein kinase) is an energy-sensing enzyme that is activated when the energy level of cells is low (ADP and AMP contents are elevated). PCG1-α is a major regulatory factor for inducing conversion of type II muscle fibers to type I.

As shown in Table 19 and Fig. 15, compared with the breast milk group (G0 group), the content of type I and type II muscle fibers and the expression amount of mTOR proteins were all significantly reduced (P < 0.05) and the contents of AMPK and PGC1-α were markedly increased (P < 0.05) in the dorsal muscle tissue of the G1 group. This means that the muscle tissue of the piglets in the G1 group was in an energy-deficient state, the cell proliferation was slowed down, and the conversion of type II muscle fibers into type I muscle fibers occurred continuously, resulting in loss of muscle mass of the body. G3 group could significantly up-regulate the content of type I and type II muscle fibers and the expression amount of mTOR protein in muscle tissue (P < 0.05), and at the same time reduce the relative content of AMPK and PGC1-α (P < 0.05). The G2 group could also up-regulate the content of type I and type II muscle fibers and the expression amount of mTOR protein in muscle tissue, and at the same time reduce the relative content of AMPK and PGC1-α. This indicates that dietary supplementation with odd-carbon fatty acids can improve the energy state of muscle tissue, promote cell proliferation and tissue development, increase the content of muscle fibers, inhibit the conversion of type II muscle fibers into type I muscle fibers, increase muscle mass, and finally promote the overall development of muscle tissue; in the composition of the odd-carbon chain fatty acids, the higher the proportion of C15, the more the energy state of the muscle tissue can be improved, cell proliferation and tissue development can be promoted, the content of muscle fibers can be increased, the conversion of type II muscle fibers into type I muscle fibers can be inhibited, muscle mass can be increased, and finally the overall development of muscle tissue can promoted.

**Table 19**

| | G0 | G1 | G2 | G3 |
|---|---|---|---|---|
| Dorsal muscle myosin heavy chain 1 (MYHC1) | 0.37 | 0.05 | 0.17 | 0.20 |
| Dorsal muscle myosin heavy chain 2a (MYHC2a) | 0.37 | 0.07 | 0.19 | 0.26 |
| Dorsal muscle myosin heavy chain 2b (MYHC2b) | 0.32 | 0.04 | 0.13 | 0.17 |
| Dorsal muscle myosin heavy chain 2x (MYHC2x) | 0.34 | 0.05 | 0.15 | 0.22 |
| Dorsal muscle mammalian target protein of rapamycin (mTOR) | 0.32 | 0.04 | 0.12 | 0.19 |
| Dorsal muscle phosphorylated AMP-activated protein kinase (p-AMPK) | 0.05 | 0.38 | 0.24 | 0.18 |
| Dorsal muscle peroxisome proliferator-activated receptor gamma coactivator 1-alpha (PGC-1α) | 0.06 | 0.33 | 0.22 | 0.20 |

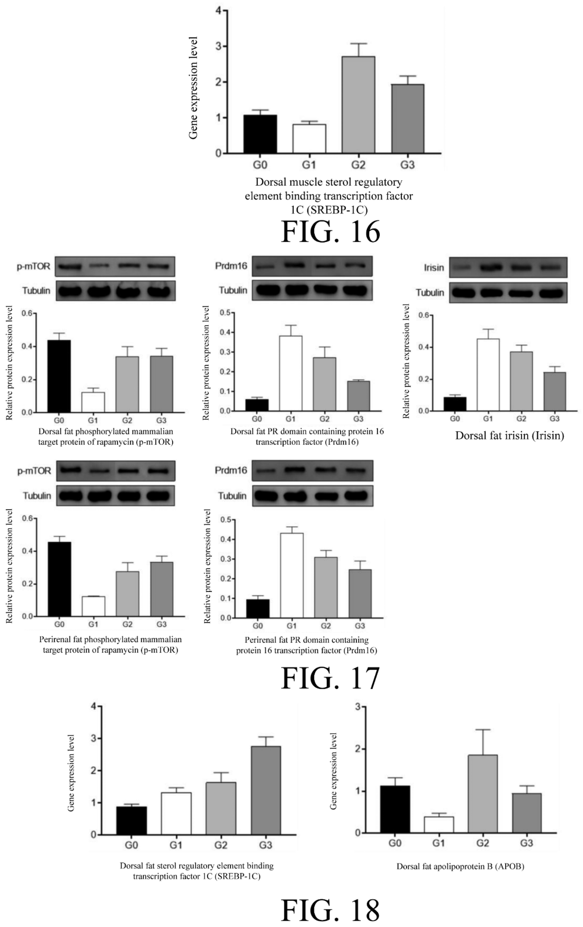

Muscle is a main target organ for energy metabolism and glycolipid metabolism, and there exist active lipid metabolism processes, especially when exercise is performed. SREBP-1 is a transcription factor associated with lipid metabolism in cells, and is closely related to lipid uptake and synthesis. As shown in Table 20 and Fig. 16, compared with the breast milk group (G0 group), the content of SREBP-1 in the G1 group decreased to some extent, but not significantly. However, the G2 group and the G3 group could significantly up-regulate the expression amount of SREBP-1 in muscle tissue, denoting that there existed more active lipid anabolism in muscle tissue. This indicates that dietary supplementation with odd-carbon fatty acids can increase the uptake and utilization of lipids by muscle tissue, improve energy state, and be beneficial to the development of muscle tissue, which can also correspond to the above-mentioned proliferation of muscle cells and increase in muscle mass.

**Table 20**

| | G0 | G1 | G2 | G3 |
|---|---|---|---|---|
| Dorsal muscle sterol regulatory element binding transcription factor 1 (SREBP-1C) | 1.08 | 0.82 | 2.73 | 1.94 |

### (6) Development and metabolism of adipose tissue

Similar to the other tissues described previously, mTOR protein is an important link in the production and maintenance of lipids in adipose tissue and the development of adipose tissue. The PR domain containing protein 16 transcription factor (Prdm16) and irisin (Irisin) can act on white adipocytes to induce their transformation into brown adipocytes. Although brown adipose tissue is present in small amounts in the body of an adult, its heat production efficiency is extremely high, and a small piece of brown adipose can burn a large amount of calories. Activated brown adipose tissue generates heat by rapidly consuming glucose and fat, and is important for combating obesity. Therefore, Prdm16 and Irisin are molecular switches for the browning of white fat, and are related to lipolysis. In early stages of animal development, a certain fat store plays an important role in the overall energy metabolism and homeostasis of the body. As shown in Table 21 and Fig. 17, compared with the breast milk group (G0 group), the content of activated mTOR protein was significantly reduced (P < 0.05) and the contents of Prdm16 and Irisin were significantly increased (P < 0.05) in the dorsal fat and perirenal fat of the piglets of the G1 group, suggesting that lipid synthesis and adipose tissue development were hindered, and the browning process of white fat was intensified, with obvious lipolysis. The G2 group and the G3 group could significantly up-regulate the expression of activated mTOR, and reduce the expression amount of Prdm16 and Irisin. This denotes that dietary supplementation with odd-carbon fatty acids can promote adipose tissue development by inhibiting lipolysis and at the same time increasing lipid synthesis; in the composition of the odd-carbon chain fatty acids, the higher the proportion of C15, the more adipose tissue development can be promoted by inhibiting lipolysis and at the same time increasing lipid synthesis.

**Table 21**

| | G0 | G1 | G2 | G3 |
|---|---|---|---|---|
| Dorsal fat mammalian target protein of rapamycin (mTOR) | 0.44 | 0.12 | 0.34 | 0.34 |
| Dorsal fat PR domain containing protein 16transcription factor (Prdm16) | 0.06 | 0.38 | 0.27 | 0.15 |
| Dorsal fat irisin (Irisin) | 0.09 | 0.45 | 0.37 | 0.24 |
| Perirenal fat mammalian target protein of rapamycin (mTOR) | 0.46 | 0.12 | 0.28 | 0.33 |
| Perirenal fat PR domain containing protein 16transcription factor (Prdm16) | 0.10 | 0.43 | 0.31 | 0.25 |

Similarly, SREBP-1 is a transcription factor associated with lipid metabolism in cells, and is closely related to lipid uptake and synthesis. APOB (apolipoprotein) is a major component of lipoproteins and is responsible for the transport of lipids in various systems. As shown in Table 22 and Fig. 18, compared with the breast milk group (G0 group), the SERBP-1 level was comparable but the APOB was significantly reduced (P < 0.05) in the piglets of the G1 group, suggesting that the transport of lipids might have been hindered. The G3 group had significantly up-regulated SERBP-1 level in the adipose tissue of the piglets, and restored the APOB level to the level of the breast milk group (P < 0.05). This means that dietary supplementation with odd-carbon fatty acids can increase the lipid synthesis in adipose tissue and enhance the transport of lipids by the body, helping to maintain the energy balance of the adipose tissue and the body.

**Table 22**

| | G0 | G1 | G2 | G3 |
|---|---|---|---|---|
| Dorsal fat sterol regulatory element binding transcription factor 1 (SREBP-1C) | 0.88 | 1.33 | 1.64 | 2.76 |
| Dorsal fat apolipoprotein B (APOB) | 1.12 | 0.40 | 1.86 | 0.95 |

### (7) Oxidation-reduction state of the body

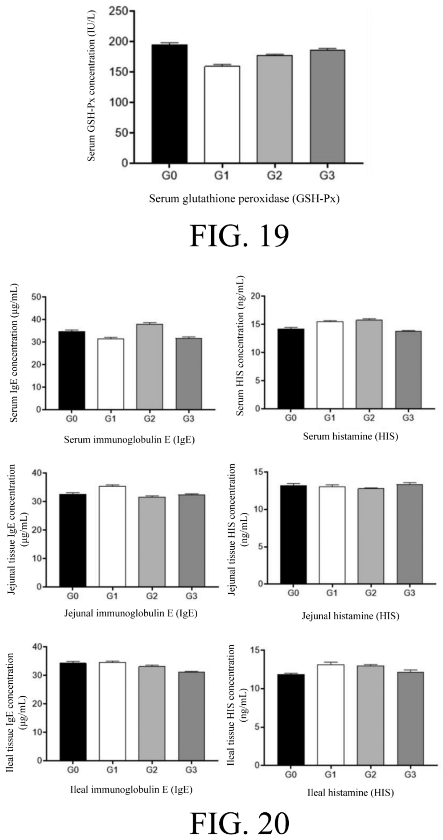

Glutathione peroxidases (GSH-PX) are a class of enzymes with peroxidase activity, and play a role in detoxification in organisms. Their main biological functions are to reduce lipid peroxides into corresponding alcohols and to reduce free hydrogen peroxide into water, which impart them with an antioxidant property. As shown in Table 23 and Fig. 19, compared with the breast milk group (G0 group), the GSH-PX content was significantly reduced in the piglets of the G1 group, suggesting that the antioxidant property of the body of the piglets was weakened, and cell structure, etc., would be easily damaged by peroxides or the like. The G2 group and the G3 group had significantly up-regulated content of GSH-PX in the serum of the piglets, restoring the content to a level close to that of the breast milk group. This means that dietary supplementation with odd-carbon fatty acids can improve the overall antioxidant capacity of the body, helping to maintain overall redox equilibrium;

**Table 23**

| | G0 | G1 | G2 | G3 |
|---|---|---|---|---|
| Serum glutathione peroxidases (GSH-Px) | 194.95 | 160.54 | 177.90 | 186.16 |

### (8) Allergic reaction in the body and the intestinal tract

The stronger an allergic reaction, the more IgE and HIS are released. As shown in Table 24 and Fig. 20, G3 had significantly reduced blood HIS (P < 0.05), jejunal IgE (P < 0.05), ileal IgE (P < 0.05) and HIS (P < 0.05) compared with G1, and the jejunal IgE and ileal IgE in the G2 group were also reduced compared with the G1 group. This indicates that, compared with even-carbon fatty acids, dietary supplementation with odd-carbon fatty acids would be beneficial to reducing an allergic reaction in the body and the jejunum and ileum.

**Table 24**

| | G0 | G1 | G2 | G3 |
|---|---|---|---|---|
| Serum immunoglobulin E (IgE) | 34.87 | 31.54 | 38.07 | 31.75 |
| Serum histamine (HIS) | 14.25 | 15.50 | 15.79 | 13.85 |
| Jejunal immunoglobulin E (IgE) | 32.66 | 35.49 | 31.65 | 32.64 |
| Jejunal histamine (HIS) | 13.22 | 13.04 | 12.97 | 13.19 |
| Ileal immunoglobulin E (IgE) | 34.36 | 34.60 | 33.14 | 31.14 |
| Ileal histamine (HIS) | 11.86 | 13.17 | 13.02 | 12.18 |

### (10) Nutritional effects

### 4.1 Growth performance

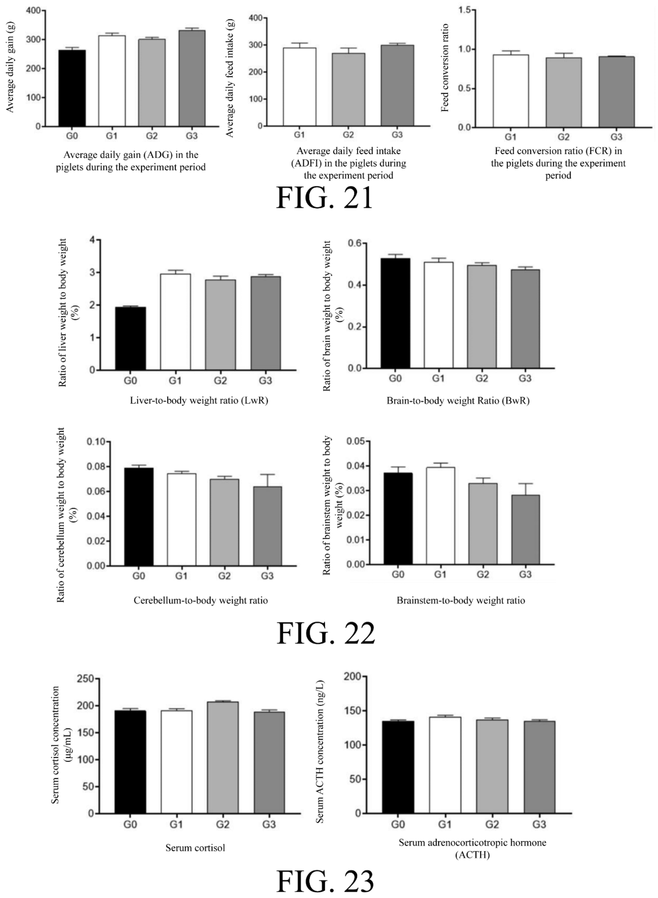

As shown in Table 25 and Fig. 21, dietary supplementation with odd-carbon fatty acids did not significantly affect the average daily gain, average daily feed intake and feed conversion ratio in the piglets.

**Table 25**

| | G0 | G1 | G2 | G3 |
|---|---|---|---|---|
| Average daily gain (ADG) in the piglets during the experiment period | 263.75 | 313.92 | 301.75 | 331.75 |
| Average daily feed intake (ADFI) in the piglets during the experiment period | | 291.06 | 270.63 | 299.81 |
| Feed conversion ratio (FCR) in the piglets during the experiment period | | 0.93 | 0.90 | 0.91 |

As shown in Table 26 and Fig. 22, dietary supplementation with odd-carbon fatty acids did not affect the weight ratios of the livers, brains, cerebella and brainstems of the piglets.

**Table 26**

| | G0 | G1 | G2 | G3 |
|---|---|---|---|---|
| Liver-to-body weight ratio (LwR) | 1.94 | 2.97 | 2.79 | 2.88 |
| Brain-to-body weight ratio (BwR) | 0.53 | 0.51 | 0.50 | 0.48 |
| Cerebellum-to-body weight ratio | 0.08 | 0.07 | 0.07 | 0.06 |
| Brainstem-to-body weight ratio | 0.04 | 0.04 | 0.03 | 0.03 |

The feces of the piglets were observed every day during the experiment period. It was found that the piglets had no obvious diarrhea and had good growth conditions throughout the whole process, indicating that dietary supplementation with odd-carbon fatty acids did not cause diarrhea in the piglets.

Nonspecific reactions in individuals caused by various tension stimulants (stressors) are stress reactions, and long-term stress reactions may lead to pathological manifestations. When the body develops a stress reaction, cortisol (Cortisol) and adrenocorticotropic hormone (ACTH) in the blood will increase. As shown in Table 27 and Fig. 23, the blood stress hormones (cortisol (Cortisol) and adrenocorticotropic hormone (ACTH)) were not significantly different between the G1 group and the G3 group (P > 0.05), and there were also no marked differences for the G2 group, indicating that dietary supplementation with odd-carbon fatty acids did not cause stress reactions in the suckling piglets.

**Table 27**

| | G0 | G1 | G2 | G3 |
|---|---|---|---|---|
| Serum cortisol (Cortisol) | 191.00 | 190.36 | 204.90 | 189.52 |
| Serum adrenocorticotropic hormone (ACTH) | 135.35 | 140.95 | 137.31 | 134.95 |

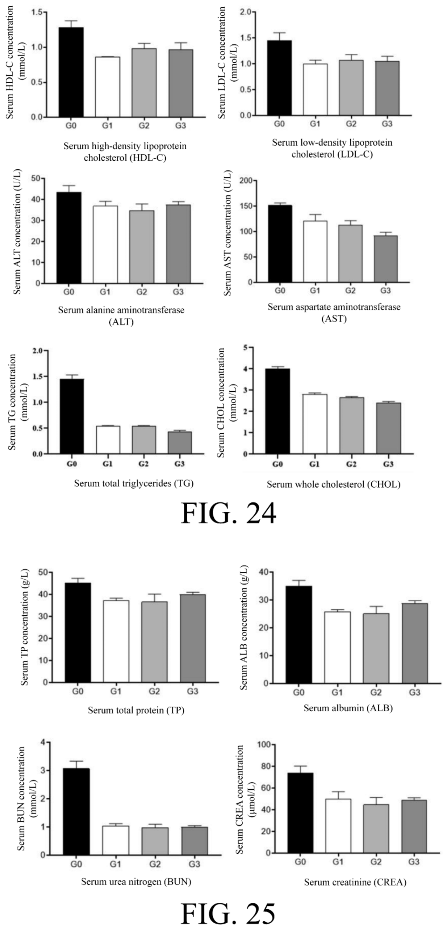

Abnormal lipid metabolism in the body may cause an increase in triglyceride (TG), cholesterol (CHOL), and low-density lipoprotein (LDL-C) and a decrease in high-density lipoprotein (HDL-C) in the blood, and the liver would be damaged (when the liver is damaged, glutamic oxalacetic transaminase (AST) and glutamic pyruvic transaminase (ALT) in the blood will increase). As shown in Table 28 and Fig. 24, there were no significant differences in TG, CHOL, HDL-C and LDL-C in the blood of the piglets in the G1 group versus the G2 group and the G3 group, and there were no significant differences in AST and ALT either. This indicates that compared with even-carbon fatty acids, dietary supplementation with odd-carbon fatty acids did not cause abnormal lipid metabolism in the body.

**Table 28**

| | G0 | G1 | G2 | G3 |
|---|---|---|---|---|
| Serum high-density lipoprotein cholesterol (HDL-C) | 1.29 | 0.86 | 0.98 | 0.97 |
| Serum low-density lipoprotein cholesterol (LDL-C) | 1.45 | 1.00 | 1.08 | 1.05 |
| Serum alanine aminotransferase (ALT) | 43.50 | 36.95 | 34.91 | 37.56 |
| Serum aspartate aminotransferase (AST) | 151.68 | 121.38 | 113.00 | 92.14 |
| Serum total triglyceride (TG) | 1.46 | 0.54 | 0.54 | 0.44 |
| Serum total cholesterol (CHOL) | 4.00 | 2.81 | 2.67 | 2.42 |

Table 29 and Fig. 25 show the effect of daily ration treatment on the indexes related to protein metabolism in the blood of the piglets. As can be seen from Fig. 25, dietary supplementation with odd-carbon fatty acids did not affect protein metabolism in the blood.

**Table 29**

| | G0 | G1 | G2 | G3 |
|---|---|---|---|---|
| Serum total protein (TP) | 45.24 | 37.23 | 36.69 | 40.03 |
| Serum albumin (ALB) | 35.08 | 25.93 | 25.14 | 28.83 |
| Serum urea nitrogen (BUN) | 3.08 | 1.04 | 0.99 | 1.00 |
| Serum creatinine (CREA) | 74.00 | 50.13 | 44.88 | 49.25 |

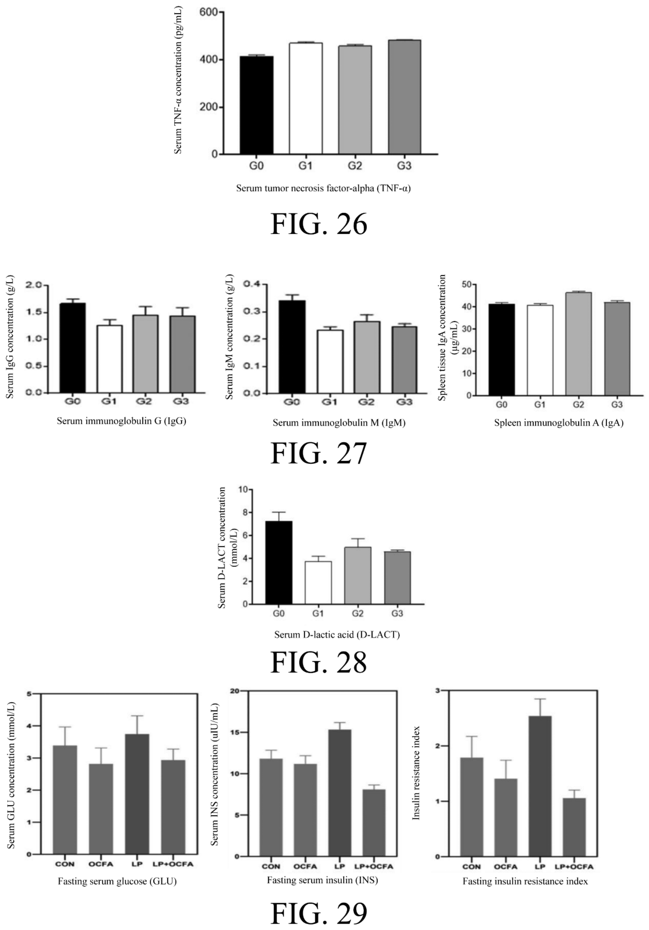

TNF-α is a cytokine involved in systemic inflammation, and is mainly secreted by macrophages. It can cause acute reactions, promote fever, and cause cell apoptosis. The content of TNF-α in serum can to some extent serve as an indexing factor that reflects the overall inflammatory condition of the body. Table 30 and Fig. 26 show the effect of daily ration treatment on the key index of serum inflammation in the piglets. As can be seen from Fig. 26, compared with the G0 and G1 groups, dietary supplementation with odd-carbon fatty acids did not impose an additional burden on the overall inflammatory condition of the piglets.

**Table 30**

| | G0 | G1 | G2 | G3 |
|---|---|---|---|---|
| Serum tumor necrosis factor (TNF-α) | 415.13 | 470.40 | 458.85 | 476.79 |

Table 31 and Fig. 27 show the effect of daily ration treatment on the immunoglobulins in the blood and spleen of the piglets. As can be seen from the figure, there were no significant differences in IgG and IgM in the blood of the piglets in the G2 group and the G3 group versus the G1 group, and there were no differences in the level of IgA in the spleen either, indicating that compared with even-carbon fatty acids, dietary supplementation with odd-carbon fatty acids did not cause significant changes in the humoral immunity of the body.

**Table 31**

| | G0 | G1 | G2 | G3 |
|---|---|---|---|---|
| Serum immunoglobulin G (IgG) | 1.67 | 1.26 | 1.45 | 1.44 |
| Serum immunoglobulin M (IgM) | 0.34 | 0.23 | 0.27 | 0.25 |
| Spleen immunoglobulin A (IgA) | 41.24 | 40.83 | 46.44 | 42.04 |

D-lactic acid (LACT) is a metabolic end product of indigenous bacteria in the gastrointestinal tract, and the body of a mammal does not have an enzyme system that rapidly metabolizes and decomposes it. Therefore, an increase in the level of D-lactic acid in the blood can reflect a change in intestinal permeability. Fig. 28 shows the effect of daily ration treatment on D-lactic acid in the blood of the piglets. As can be seen from Table 32 and Fig. 28, there were no significant differences in D-lactic acid in the serum of the piglets in the G2 group and the G3 group versus G1, indicating that compared with even-carbon fatty acids, dietary supplementation with odd-carbon fatty acids did not cause a significant change in the intestinal permeability of the body.

**Table 32**

| | G0 | G1 | G2 | G3 |
|---|---|---|---|---|
| Serum D-lactic acid (D-LACT) | 7.26 | 3.76 | 4.99 | 4.60 |

### Example 2

### 1. Basic experimental procedure

In this experiment, female mice in the gestation period were fed with normal daily ration and low-protein daily ration (an abnormal diet) using OCFA-containing oil or fat and non-OCFA-containing oil or fat, respectively (blending with Hard ST and high-oleic sunflower seed oil to eliminate the effects of DHA, the ratio of SFA to UFA, and the ratio of n-3 unsaturated fatty acid to n-6 unsaturated fatty acid in OCFA algal oil). The female mice were intragastrically fed on the 7.5 day of gestation. The female mice were fasted for 6 h starting from the 18.5 day of the gestation period, and were then anesthetized with ether, and killed for sampling.

The various determination methods of this example were the same as those of Example 1, unless otherwise specified.

### 2. Preparation of oil or fat

Oil or Fat 1: DHA algal oil (Qingdao Keyuan, obtained by refining the purchased crude oil, reference was made to "Optimization of the refining processes for microalgae DHA" by Huang Huaixin for the refining method, and the batch of the crude oil was MKD120056) and Hard ST(Yihai Kerry, the same batch of product as the Hard ST used in Example 1) were melted and mixed uniformly in a mass ratio of 1:1.2. Then, the obtained mixed oil and corn oil were melted and mixed uniformly in a mass ratio of 2:3 to obtain Oil or Fat 1, thereby reducing the melting point of the oil or fat to facilitate intragastric feeding.

Oil or Fat 2: OCFA algae oil (Yihai Kerry) and high-oleic sunflower seed oil (Yihai Kerry) were melted and mixed uniformly in a mass ratio of 1:0.1. Then, the obtained mixed oil and corn oil were melted and mixed uniformly in a mass ratio of 2:3 to obtain Oil or Fat 2, thereby reducing the melting point of the oil or fat. (The animal experiments in the present application used the oil or fat obtained by this preparation method)

Oil or Fat 2 could also be prepared according to the following method: The glyceryl tritridecanoate, glyceryl tri-pentadecanoate and glyceryl tri-heptadecanoate prepared according to the method described in Example 1 and DHA algal oil (Qingdao Keyuan, obtained by refining purchased crude oil, reference was made to "Optimization of the refining processes for microalgae DHA" by Huang Huaixin for the refining method, and the batch of the crude oil was MKD120056) and high-oleic sunflower seed oil (Yihai Kerry, the same batch of product as the high-oleic sunflower seed oil used in Example 1) were melted and mixed uniformly in a mass ratio of 1:46:10:41:2. Then, the obtained mixed oil and corn oil were melted and mixed uniformly in a mass ratio of 2:3 to obtain Oil or Fat 2, thereby reducing the melting point of the oil or fat to facilitate intragastric feeding.

### 3. Fatty acid composition of the oils or fats

The oil or fat fatty acid composition of Oils or Fats 1 and 2 was determined with reference to the third method, i.e., "normalization method" in "GB5009.168-2016 Food Safety National Standard Determination of Fatty Acids in Foods". The results are as shown in Table 33.

**Table 33: Main fatty acid compositions of oils or fats**

| Fatty acid | Oil or Fat 1 | Oil or Fat 2 |
|---|---|---|
| C12:0 | 0.1 | 0.0 |
| **C13:0** | **0.0** | **0.3** |
| C14:0 | 0.8 | 0.4 |
| **C15:0** | **0.0** | **18.7** |
| C16:0 | 33.7 | 12.1 |
| C16:1 | 0.1 | 0.1 |
| **C17:0** | **0.1** | **4.0** |
| C18:0 | 2.5 | 1.4 |
| C18:1n9c | 17.8 | 17.9 |
| C18:2n6c | 34.0 | 34.3 |
| C18:3n3 | 0.6 | 0.7 |
| C20:0 | 0.3 | 0.3 |
| C20:1 | 0.2 | 0.2 |
| C20:2 | 0.0 | 0.1 |
| C20:3n6 | 0.1 | 0.1 |
| C20:4n6 | 0.0 | 0.1 |
| C22:0 | 0.1 | 0.1 |
| C20:5n3 | 0.3 | 0.2 |
| C24:0 | 0.1 | 0.1 |
| C24:1 | 0.0 | 0.1 |
| C22:6n3 | 9.2 | 9.1 |
| SUM | 100.0 | 100.0 |
| OCFA | 0.1 | 23.0 |
| SFA | 37.6 | 37.3 |
| UFA | 62.4 | 62.7 |
| SFA / UFA | 0.6 | 0.6 |
| n-3 PUFA | 10.1 | 10.0 |
| n-6 PUFA | 34.2 | 34.4 |
| n-3 PUFA / n-6 PUFA | 0.3 | 0.3 |

### 4. Animal experiments

Experimental animals: Twenty four pregnant female mice were selected for the experiments, and a 2x2 factorial experiment design was used. On day 7.5 of the gestation period, the female mice were divided into 4 treatment groups, with 3 replicates in each group, and 2 female mice in each replicate: 1) CON group; 2) OCFA group; 3) LP group; and 4) LP + OCFA group. Starting from day 7.5 of the gestation period of the female mice, the CON group and the OCFA group were fed with a normal diet with a protein level of 19%, and were intragastrically fed with oil or fat once a day, the dose for the intragastric feeding with oil or fat being 1 g/kg/day. The LP group and the LP + OCFA group were fed with a low-protein diet with a protein level of 8%, and were intragastrically fed with oil or fat once a day, the dose for the intragastric feeding with oil or fat being 1 g/kg/day. The daily ration and the intragastrically fed oil or fat for each group were shown in Table 34. The female mice were fasted for 6 h starting from the 18.5 day of the gestation period, and then sacrificed for sampling for determination and analysis. All related operation procedures met the requirements of the Ethical Committee for Animal Welfare and Animal Experiments of China Agricultural University.

**Table 34: Grouping for experimental treatment**

| Grouping | Ration | Oil or fat for intragastrically feeding |
|---|---|---|
| CON group | Normal diet | Oil or Fat 1 |
| OCFA group | Normal diet | Oil or Fat 2 |
| LP group | Low-protein diet | Oil or Fat 1 |
| LP + OCFA group | Low-protein diet | Oil or Fat 2 |

### 5. Results

### (1) Influence on glycometabolism (improving insulin resistance)

During pregnancy, the placenta produces a lot of substances, including many hormones and cytokines, which may result in the development of insulin resistance, possibly affecting the action of insulin. Insulin resistance becomes evident in the middle period of pregnancy, and most pregnant women may show insulin resistance during this process. The body can control blood glucose well by producing more insulin. However, for a small number of pregnant women, although insulin production is also increased, the amount is not sufficient, which eventually leads to gestational diabetes. Pregnant women with gestational diabetes are prone to having fetal macrosomia, fetal malformation, premature birth, premature rupture of fetal membrane, polyhydramnios, etc. In severe cases, an increase in fetal death rate may occur. The fasting serum glucose (GLU, mmol/L, determined using a biochemical analyzer), fasting insulin (INS, µIU/mL, ELISA assay) and fasting insulin resistance index (HOMA-IR = fasting serum glucose*fasting insulin/22.5; for the calculation method, reference was made to Linder, Katarzyna, et al. "Relationships of body composition and liver fat content with insulin resistance in obesity-matched estrogens and adults." Obesity 22.5 (2014): 1325 to 1331.) were determined. The results are as shown in Table 35 and Fig. 29. As can be seen from Fig. 29, compared with the CON group, the insulin resistance index of the pregnant mice in the OCFA group had a decreasing trend. Compared with the LP group, the insulin resistance index of the pregnant mice in the LP + OCFA group was significantly reduced. The above results indicate that odd-carbon fatty acids would be beneficial to controlling blood glucose during pregnancy by reducing insulin resistance during pregnancy. Especially, odd-carbon fatty acids would be beneficial to improving insulin resistance resulting from a bad diet (low-protein diet). Odd-carbon fatty acids may be beneficial to ameliorating type 2 diabetes.

**Table 35**

| | CON | OCFA | LP | LP+OCFA |
|---|---|---|---|---|
| Fasting serum glucose (GLU) | 3.39 | 2.81 | 3.75 | 2.93 |
| Fasting serum insulin (INS) | 11.81 | 11.18 | 15.33 | 8.09 |
| Fasting insulin resistance index | 1.79 | 1.41 | 2.55 | 1.05 |

### (2) Influence on bile acid metabolism (lowering the level of serum bile acid)

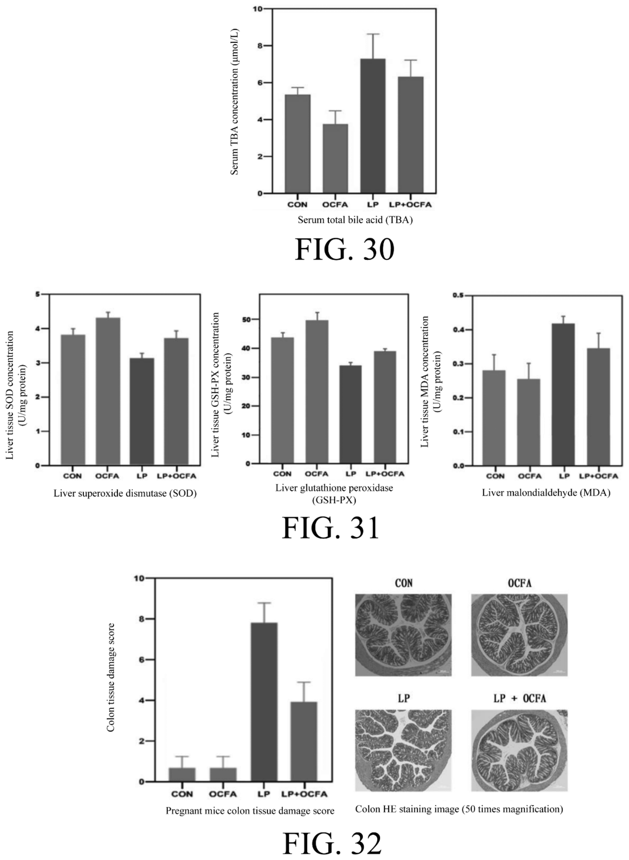

Gestational intrahepatic cholestasis is a disease specific to the gestation period, with an increase in serum bile acids (TBA, determined using a biochemical analyzer) as a specific index. This complication can cause an increase in fetal distress, premature labor, and perinatal mortality rate. As can be seen from Table 36 and Fig. 30, compared with the CON group, serum bile acids in the pregnant mice in the OCFA group were significantly reduced. The result showed that odd-carbon fatty acids would have the effect of reducing serum bile acids in pregnant women. Compared with the CON group, the level of serum bile acids in the pregnant mice in the LP group was significantly increased, indicating that the poor diet caused abnormal serum bile acids in the pregnant mice, which would not be beneficial to fetal health. After supplementation with odd-carbon fatty acids (LP + OCFA group), the serum bile acids in the pregnant mice had a decreasing trend. In general, gestational intrahepatic cholestasis is mainly dealt with by using hepatoprotective drugs. Therefore, a person skilled in the art can envisage that use of odd-carbon fatty acids in combination with hepatoprotective drugs should have the effect of improving gestational intrahepatic cholestasis resulting from poor diet or disease.

**Table 36**

| | CON | OCFA | LP | LP+OCFA |
|---|---|---|---|---|
| Serum total bile acid (TBA) | 5.36 | 3.76 | 7.31 | 6.33 |

### (3) Oxidative stress in the liver

Superoxide dismutase (SOD) is the most important and optimal free radical scavenger in organisms. Glutathione peroxidase (GSH-PX) is an important peroxide-degrading enzyme widely present in organisms, which can reduce toxic peroxides into non-toxic hydroxyl compounds, thereby protecting the structure and function of cell membranes from being interfered with and damaged by peroxides. Malondialdehyde (MDA) is a membrane lipid peroxidation product, and the amount of thereof can directly reflect the level of peroxidation of cell membranes. Excessive accumulation of malondialdehyde will cause cross-linking and polymerization of macromolecules of life such as proteins and nucleic acids, resulting in changes in the structure and function of cell membranes. Table 37 and Fig. 31 show the effect of daily ration and oil or fat treatment on the oxidative stress in the livers of pregnant mice. Fig. 31 shows that compared with the CON group, the hepatic superoxide dismutase (SOD) and glutathione peroxidase (GSH-PX) were significantly increased and the content of malondialdehyde (MDA) was decreased in the livers of the pregnant mice in the OCFA group. This indicates that odd-carbon fatty acids can have the effect of reducing oxidative stress in the livers of mammals with normal diet during pregnancy, thus improving the body's antioxidant capacity. Compared with the CON group, the superoxide dismutase (SOD) and glutathione peroxidase (GSH-PX) were significantly decreased and the malondialdehyde (MDA) was significantly increased in the LP group, indicating that the low-protein diet caused oxidative stress in the livers of the pregnant mice. However, compared with the LP group, the hepatic superoxide dismutase (SOD) and glutathione peroxidase (GSH-PX) were significantly increased and the malondialdehyde (MDA) was significantly decreased in the livers of the pregnant mice of the LP + OCFA group. This indicates that odd-carbon fatty acids have the effect of reducing oxidative stress in mammals during pregnancy resulting from an abnormal diet, thus improving the body's antioxidant capacity. The above results all show that supplementation with odd-carbon fatty acids can reduce the oxidative stress in the livers of mammals during pregnancy.

**Table 37**

| | CON | OCFA | LP | LP+OCFA |
|---|---|---|---|---|
| Liver superoxide dismutase (SOD) | 3.91 | 4.43 | 3.14 | 3.74 |
| Liver glutathione peroxidase (GSH-Px) | 44.11 | 50.16 | 34.28 | 39.30 |
| Liver malondialdehyde (MDA) | 0.29 | 0.25 | 0.42 | 0.34 |

### (4) Colon health

### (I) Histological score

The scoring method is: The degree of epithelial cell damage, the degree of crypt damage and the degree of inflammatory cell infiltration were observed under a microscope, and the three degrees were scored separately. The sum of the three scores was the final score of the scoring. The results are as shown in Table 38. For the evaluation method, reference was made to Kim, Janice J., et al. "Investigating intestinal inflammation in DSS-induced model of IBD." Journal of visualized experiments: JoVE 60 (2012).

Table 38 and the results of colonic HE staining in Fig. 32 showed that the structure of the colons of the LP group underwent a marked change, such as epithelial damage, compared with the CON group, while the colons in the LP + OCFA group exhibited damage repair, and was closer to that of the CON group. The significant increase in the histological scores of the colons of the pregnant mice in the LP group compared with the CON group indicates that the low-protein diet caused histopathological damage to the colonic tissue. The significant decrease in the histological score of the colon of the pregnant mice in the LP+OCFA group compared with the LP group indicates that odd-carbon fatty acids had the effect of improving the histopathological damage of the intestinal tract induced by an abnormal diet or a disease.

**Table 38**

| | CON | OCFA | LP | LP+OCFA |
|---|---|---|---|---|
| Score of damage to the colonic tissue of pregnant mice | 0.67 | 0.67 | 7.83 | 4.00 |

### (ii) Inflammations of the intestinal tract

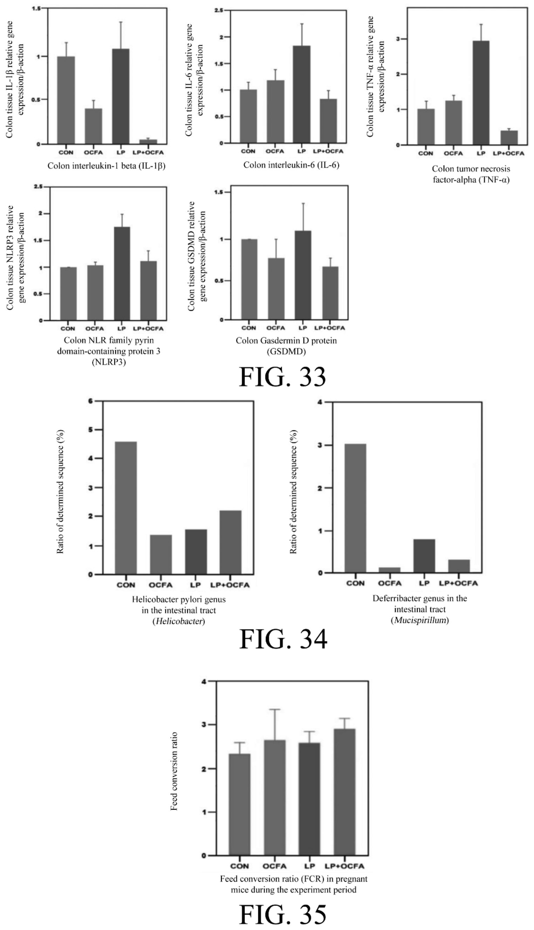

Table 39 and Fig. 33 show the effect of daily ration and oil or fat treatment on the gene expression level of five major inflammatory factors (IL-1β, IL-6, TNF-α, NLRP3 and GSDMD, determined by quantitative PCR) in the colons of pregnant mice. As shown in Fig. 33, in the normal diet group, dietary supplementation with odd-carbon fatty acids (OCFA group) reduced the expression amounts of IL-1β and GSDMD in colonic tissue, suggesting that odd-carbon fatty acids could alleviate inflammatory conditions. Compared with the CON group, feeding with a low-protein diet (the LP group) significantly up-regulated the expression amounts of the foregoing five inflammatory factors in the colon, indicating that the low-protein diet exacerbated the inflammatory reactions in the colon. However, after the mice fed with the low-protein ration were supplemented with odd-carbon fatty acids (the LP + OCFA group), the levels of the inflammatory factors all dropped to the levels of the control group, or even lower. This indicates that odd-carbon fatty acids can play a positive role in alleviating inflammations in the intestinal tract. In addition, it has been reported in the literature that a high incidence of colorectal cancer is associated with some inflammations in the intestinal tract. Therefore, a person skilled in the art can, on the basis of the above experimental results, envisage that odd-carbon fatty acids may have a positive effect on inflammatory bowel disease and colorectal cancer.

**Table 39**

| | CON | OCFA | LP | LP+OCFA |
|---|---|---|---|---|
| Colon interleukin-1 beta (IL-1β) | 1.01 | 0.41 | 1.10 | 0.06 |
| Colon interleukin-6 (IL-6) | 1.01 | 1.19 | 1.90 | 0.82 |
| Colon tumor necrosis factor-alpha (TNF-α) | 1.02 | 1.24 | 2.92 | 0.40 |
| Colon NLR family pyrin domain-containing 3 protein (NLRP3) | 1.00 | 1.04 | 1.75 | 1.12 |
| Colon Gasdermin D protein (GSDMD) | 1.00 | 0.77 | 1.09 | 0.65 |

### (iii) Microecology in the intestinal tract

Table 40 and Fig. 34 show the effect of daily ration and oil or fat treatment on two harmful bacteria in the intestinal tracts of pregnant mice. A large number of studies have shown that the Helicobacter pylori genus and the Deferribacter genus can induce inflammation in the intestinal tract. Fig. 34 shows that for pregnant mice fed with a normal diet, supplementation with odd-carbon fatty acids (the OCFA group) significantly reduced the abundance of microorganisms of the Helicobacter pylori genus and the Deferribacter genus in the feces of the pregnant mice (The fecal samples of the pregnant mice on the day of sacrifice were taken for DNA extraction, and16S rRNA gene sequencing was performed; Shanghai Majorbio was entrusted to perform the DNA extraction and determination). In the mice fed with the low-protein diet, supplementation with odd-carbon fatty acids (the LP + OCFA group) also reduced the abundance of the microorganisms of the Deferribacter genus in the intestinal tract. This indicates that odd-carbon fatty acids may have the effect of improving the microecology in the intestinal tract.

**Table 40**

| | CON | OCFA | LP | LP+OCFA |
|---|---|---|---|---|
| Helicobacter pylori genus (Helicobacter) in the intestinal tract | 4.78 | 1.44 | 1.63 | 2.30 |
| Deferribacter genus (Mucispirillum) in the intestinal tract | 3.14 | 0.13 | 0.81 | 0.31 |

Helicobacter pylori is a common human pathogenic bacterium, infecting about half of the world's population. Helicobacter pylori infection can cause a series of gastrointestinal diseases of different degrees, such as chronic gastritis, dyspepsia, and peptic ulcer. Moreover, Helicobacter pylori is also a definite human carcinogen, and is an important cause of gastric cancer. Eradication therapy in patients infected with Helicobacter pylori can not only improve the gastrointestinal diseases associated with Helicobacter pylori, but also effectively reduce the risk of affliction with gastric cancer.

Antibiotics represent a current conventional method for the eradication therapy of Helicobacter pylori. However, with the gradual increase in the antibiotic resistance of Helicobacter pylori worldwide, the success rate of the eradication therapy with antibiotics is continuously decreasing. Moreover, with the rise of research on the microbiome of the intestinal tract, the potential adverse effects of antibiotic treatment (especially repeated and long-term use of antibiotics) on the microecology in the intestinal tract have also attracted extensive attention from the academic community. To address these existing problems and challenges, researchers have begun to look for other auxiliary and substitutive therapies.

The results of the present invention suggest that odd-carbon fatty acids may find use in substitutive or auxiliary methods for eradicating or treating Helicobacter pylori.

### (5) Nutritional effects

The ratio of the amount of feed used divided by the increment in the body weight is feed-to-meat ratio, also known as feed conversion ratio. As can be seen from Table 41 and Fig. 35, compared with the CON group, the OCFA group had an increasing trend of feed conversion ratio. Also, compared to the LP group, the LP + OCFA group had an increasing trend of feed conversion ratio. This indicates that odd-carbon fatty acids are beneficial for controlling the body weight of mammals during pregnancy.

**Table 41**

| | CON | OCFA | LP | LP+OCFA |
|---|---|---|---|---|
| Feed conversion ratio (FCR) in pregnant mice during the experiment period | 2.38 | 2.65 | 2.57 | 2.96 |

Table 42 and Fig. 36 show the effect of daily ration and oil or fat treatment on the lipid metabolism of pregnant mice (determined using a blood biochemical kit). Fig. 36 shows that compared with the CON group, the serum total cholesterol, triglyceride, HDL and LDL in the low LP group all showed a significant decrease, indicating that the low-protein diet caused abnormal lipid metabolism in the pregnant mice; compared with the LP group, the LP + OCFA group did not cause a more significant decrease in the foregoing indexes, indicating that dietary supplementation with odd-carbon fatty acids did not adversely affect the lipid metabolism in mammals during pregnancy. Moreover, in the figure, the serum high-density lipoprotein (HDL) was markedly increased in the OCFA group compared with the CON group, and in the LP + OCFA group compared with the LP group. The high-density lipoprotein is an anti-atherosclerotic plasma lipoprotein and is a protective factor for coronary heart disease. The above results show that odd-carbon fatty acids can increase the serum high-density lipoprotein, improve atherosclerosis and reduce the risk of coronary heart disease.

**Table 42**

| | CON | OCFA | LP | LP+OCFA |
|---|---|---|---|---|
| Serum total triglyceride (TG) | 2.85 | 2.95 | 1.08 | 0.89 |
| Serum high-density lipoprotein (HDL) | 0.84 | 0.93 | 0.49 | 0.61 |
| Serum low-density lipoprotein (LDL) | 0.32 | 0.31 | 0.22 | 0.26 |

From the foregoing, it will be appreciated that although specific embodiments of the present invention have been described for the purpose of illustrative description, it should be understood that the scope of the present invention is not limited to these specific embodiments. Various variations or improvements may be made to the present invention by a person skilled in the art without departing from the spirit and scope of the present invention. These variations or improvements all fall within the scope of protection of the present invention.

## Claims

1. A use of odd-carbon fatty acids in the preparation of a formulation or medicament for one or more of the following purposes:
(1) increasing the protein expression abundance of a tight junction-associated protein (such as Occludin, Claudin and/or ZO-1) in a subject; especially increasing the protein expression abundance of the tight junction-associated protein (e.g. Occludin, Claudin and/or ZO-1) in the intestinal tract (e.g. mucosa of the intestinal tract), and more specifically the protein expression abundance of the tight junction-associated protein (e.g. Occludin, Claudin and/or ZO-1) in the duodenum, jejunum, and ileum (e.g. mucosa thereof);
(2) reducing the content of a corticotropin-releasing hormone (CRF) in a tissue of the intestinal tract of a subject; and more preferably, reducing the content of the corticotropin-releasing hormone (CRF) in a tissue of the ileum;
(3) improving or enhancing the mechanical barrier of the intestinal tract of a subject; wherein improving the mechanical barrier of the intestinal tract can protect against the invasion of bacteria, toxins and foreign antigen substances to the body, maintain the stability of the body's internal environment, and create a basis for nutrient absorption;
(4) increasing the content of an immunoglobulin (e.g., IgG, IgA and/or IgM) in the intestinal tract of a subject; preferably, increasing the content of an immunoglobulin (such as IgG, IgA and/or IgM) in the jejunum and ileum; more preferably, increasing the content of immunoglobulin IgG, IgA and/or IgM in the jejunum; and more preferably, increasing the content of immunoglobulin A in the ileum;
(5) increasing the protein expression abundance of TLR4 and NF-κB in a tissue of the intestinal tract, especially in the duodenum, jejunum, and ileum, of a subject;
(6) maintaining the homeostasis of an ileal inflammatory cytokine (e.g., TNF-α, IL-1β IL-6, and/or IL-10) in a subject;
(7) increasing the expression amount of ileal mucin 2 in a subject;
(8) increasing the expression of HIF-1α in the intestinal tract, and promoting the survival of cells of the intestinal tract in the hypoxic environment of the intestinal tract;
(9) maintaining immune homeostasis in the intestinal tract (especially the ileum) of a subject;
(10) increasing the content of the nuclear protein Ki-67 in a tissue of the intestinal tract of a subject, the tissue of the intestinal tract preferably being the duodenum, jejunum and/or ileum; and promoting the proliferation of intestinal epithelial cells in the subject;
(11) activating the mTOR signaling pathway in the intestinal tract of a subject, and increasing the expression amount of an activated mTOR protein in cells of the intestinal tract (especially cells of the small intestine, such as the duodenum, jejunum, and ileum) and/or increasing the level of an activated S6 kinase in cells of the intestinal tract (especially cells of the small intestine, such as the duodenum, jejunum, and ileum);
(12) increasing the level of digestive enzymes (including but not limited to maltase, aminopeptidase and/or sucrase) in the intestinal tract, especially the small intestine, of a subject, and promoting the absorption of nutrients;
(13) increasing the content of 5-hydroxytryptamine in a tissue of the intestinal tract (especially the duodenum, jejunum and/or ileum) of a subject and the content of Substance P (a neuropeptide) in the ileum, and enhancing the function of the small intestine, such as increasing intestinal peristalsis, promoting the contraction of the smooth muscle of the gastrointestinal tract, inhibiting the secretion of gastric acid and bile, regulating local gastrointestinal blood flow, etc.;
(14) promoting the development of the intestinal tract in a subject;
(15) down-regulating the expression amount of a mitochondrial autophagy-associated protein (e.g., ULK1, TFEB, and/or Parkin) in cells of the intestinal tract (especially the duodenum, jejunum and/or ileum) of a subject, and alleviating mitochondrial dysfunction;
(16) up-regulating the level of a lipid synthesis-associated transcription factor (such as PPARγ and/or SREBP-1) in the intestinal tract (especially the small intestine, and especially the duodenum and jejunum) of a subject, and increasing the absorption and anabolism of lipids in the intestinal tract (especially the small intestine, and especially the duodenum and jejunum);
(17) maintaining the energy homeostasis of cells of the intestinal tract of a subject, and improving the energy metabolism in the intestinal tract;
(18) up-regulating the content of type I and type II muscle fibers and the expression amount of an mTOR protein in muscle tissue of a subject, and at the same time reducing the relative content of AMPK and PGC1-α;
(19) improving the energy state of muscle tissue of a subject, promoting the growth and development of cells, increasing the content of muscle fibers, inhibiting the conversion of type II muscle fibers into type I muscle fibers, increasing muscle mass, and promoting the overall development of muscle tissue;
(20) up-regulating the expression amount of SREBP-1 in muscle tissue of a subject, promoting lipid anabolism, increasing the uptake and utilization of lipids by muscle tissue, improving energy state, and promoting the development and metabolism of muscle tissue;
(21) promoting the development and metabolism of muscle tissue of a subject;
(22) up-regulating the expression of an activated mTOR in a subject, reducing the expression amount of Prdm16 and Irisin, inhibiting lipolysis, and increasing lipid synthesis to promote the development of adipose tissue;
(23) up-regulating the level of SERBP-1 and APOB in adipose tissue of a subject, increasing lipid synthesis in adipose tissue, enhancing transport of lipids by the body, and maintaining the energy balance of adipose tissue and the body;
(24) promoting the development and metabolism of adipose tissue in a subject;
(25) up-regulating the content of GSH-PX in serum of a subject, improving the overall antioxidant capacity of the body, and maintaining overall redox equilibrium;
(26) reducing the content of blood HIS, jejunal IgE, ileal IgE, jejunal HIS, and ileal HIS in a subject, and reducing an allergic reaction in the body, and the jejunum and ileum;
(27) reducing an allergic reaction in the body and intestinal tract of a subject;
(28) reducing insulin resistance in a subject during pregnancy;
(29) controlling blood sugar in a subject during pregnancy;
(30) improving insulin resistance resulting from a poor diet (e.g., a low-protein diet);
(31) preventing gestational diabetes, and preventing fetal macrosomia, fetal malformation, premature birth, premature rupture of fetal membrane and/or polyhydramnios resulting from gestational diabetes, reducing fetal death rate;
(32) reducing the level of serum bile acids in a subject during pregnancy;
(33) preventing or improving gestational intrahepatic cholestasis in a subject, such as gestational intrahepatic cholestasis resulting from poor diet or disease; and reducing the occurrence or risk of occurrence of fetal distress, premature labor, and perinatal mortality rate associated with gestational intrahepatic cholestasis in the subject;
(34) increasing the level of superoxide dismutase and glutathione peroxidase in the liver of a subject, reducing the level of malondialdehyde in the liver of the subject, and reducing oxidative stress in the subject during pregnancy;
(35) improving the pathological damaging effect on a tissue of the intestinal tract induced by an abnormal diet or a disease in a subject;
(36) reducing a colonic inflammatory factor (such as IL-1β, IL-6, TNF-α, NLRP3, and/or GSDMD) in a subject, and alleviating inflammation in the intestinal tract;
(37) treating or preventing inflammatory bowel disease and colorectal cancer;
(38) reducing the abundance of the Helicobacter pylori genus and/or Deferribacter genus in the body of a subject, and improving the microecology in the intestinal tract; and
(39) preventing or treating a disease resulting from a bacterium of the Helicobacter pylori genus in a subject, including gastric cancer.

2. The use according to claim 1, wherein the use comprises use of odd-carbon fatty acids in the preparation of a formulation or medicament for one or more of the following purposes:
(1) increasing the protein expression abundance of a tight junction-associated protein (such as Occludin, Claudin and/or ZO-1) in a subject; especially, increasing the protein expression abundance of the tight junction-associated protein (e.g. Occludin, Claudin and/or ZO-1) in the intestinal tract (e.g. mucosa of the intestinal tract), and more specifically the protein expression abundance of the tight junction-associated protein (e.g. Occludin, Claudin and/or ZO-1) in the duodenum, jejunum, and ileum (e.g. mucosa thereof); and (2) reducing the content of a corticotropic hormone-releasing hormone (CRF) in a tissue of the intestinal tract of the subject; thereby improving or enhancing the mechanical barrier of the intestinal tract of the subject; and/or
(4) increasing the content of an immunoglobulin (e.g., IgG, IgA and/or IgM) in the intestinal tract of a subject; preferably, increasing the content of an immunoglobulin (such as IgG, IgA and/or IgM) in the jejunum and ileum; more preferably, increasing the content of immunoglobulin IgG, IgA, and/or IgM in the jejunum, and increasing the content of immunoglobulin A in the ileum; (5) increasing the protein expression abundance of TLR4 and NF-κB in a tissue of the intestinal tract, especially in the duodenum, jejunum and ileum, of the subject; (6) maintaining the homeostasis of an ileal inflammatory cytokine (e.g., TNF-α, IL-1β, IL-6, and/or IL-10) in the subject; (7) increasing the expression amount of ileal mucin 2 in the subject; and (8) increasing the expression of HIF-1α in the intestinal tract, and promoting the survival of cells of the intestinal tract in the hypoxic environment of the intestinal tract; thereby maintaining immune homeostasis in the intestinal tract (especially the ileum) of the subject; and/or
(10) increasing the content of the nuclear protein Ki-67 in a tissue of the intestinal tract of a subject, and promoting the proliferation of intestinal epithelial cells of the subject; the tissue of the intestinal tract preferably being the duodenum, jejunum and/or ileum; (11) activating the mTOR signaling pathway in the intestinal tract of the subject, increasing the expression amount of an activated mTOR protein in cells of the intestinal tract (especially cells of the small intestine) and/or increasing the level of an activated S6 kinase in cells of the intestinal tract (especially cells of the small intestine); (12) increasing the level of digestive enzymes (including but not limited to maltase, aminopeptidase and/or sucrase) in the intestinal tract, especially the small intestine, of the subject, and promoting the absorption of nutrients; and (13) increasing the content of 5-hydroxytryptamine in a tissue of the intestinal tract (especially the duodenum, jejunum and/or ileum) of the subject and the content of Substance P (a neuropeptide) in the ileum, and enhancing the function of the small intestine such as increasing intestinal peristalsis, promoting the contraction of the smooth muscle of the gastrointestinal tract, inhibiting the secretion of gastric acid and bile, regulating local gastrointestinal blood flow, etc.; thereby promoting the development of the intestinal tract in the subject; and/or
(15) down-regulating the expression amount of a mitochondrial autophagy-associated protein (e.g., ULK1, TFEB, and/or Parkin) in cells of the intestinal tract of a subject, and alleviating mitochondrial dysfunction; and (16) up-regulating the level of a lipid synthesis-associated transcription factor (such as PPARγ and/or SREBP-1) in the intestinal tract (especially the small intestine) of the subject, and increasing the absorption and anabolism of lipids in the intestinal tract (especially the small intestine, and especially the duodenum and jejunum); thereby maintaining the energy homeostasis of cells of the intestinal tract of the subject, and improving the energy metabolism in the intestinal tract; and/or
(18) up-regulating the content of type I and type II muscle fibers and the expression amount of an mTOR protein in muscle tissue of a subject, and at the same time reducing the relative content of AMPK and PGC1-α; (19) improving the energy state of muscle tissue of the subject, promoting cell growth and tissue development, increasing the content of muscle fibers, inhibiting the conversion of type II muscle fibers into type I muscle fibers, increasing muscle mass, and promoting the overall development of muscle tissue; and (20) up-regulating the expression amount of SREBP-1 in muscle tissue of the subject, promoting lipid anabolism, increasing the uptake and utilization of lipids by muscle tissue, improving energy state, and promoting the development and metabolism of muscle tissue; thereby promoting the development and metabolism of muscle tissue in the subject; and/or
(22) up-regulating the expression of an activated mTOR in a subject, reducing the expression amount of Prdm16 and Irisin, inhibiting lipolysis, and increasing lipid synthesis to promote the development of adipose tissue; and (23) up-regulating the level of SERBP-1 and APOB in adipose tissue of the subject, increasing lipid synthesis in adipose tissue, enhancing transport of lipids by the body, and maintaining the energy balance of adipose tissue and the body; thereby promoting the development and metabolism of adipose tissue in the subject; and/or
(25) up-regulating the content of GSH-PX in serum of a subject, improving the overall antioxidant capacity of the body, and maintaining overall redox equilibrium; and (26) reducing the content of blood HIS, jejunal IgE, ileal IgE, jejunal HIS, and ileal HIS in the subject, and reducing an allergic reaction in the body and the jejunum and ileum; thereby reducing an allergic reaction in the body and intestinal tract of the subject; and/or
(28) reducing insulin resistance in a subject during pregnancy; and (29) controlling blood sugar of the subject during pregnancy; thereby preventing gestational diabetes, and preventing fetal macrosomia, fetal malformation, premature birth, premature rupture of fetal membrane and/or polyhydramnios resulting from gestational diabetes, and reducing fetal death rate; and/or
(32) reducing the level of serum bile acid in a subject during pregnancy, thereby preventing or improving gestational intrahepatic cholestasis in the subject, such as gestational intrahepatic cholestasis resulting from poor diet or disease; and/or, (33) reducing the occurrence or risk of occurrence of fetal distress, premature labor, and perinatal mortality rate associated with gestational intrahepatic cholestasis in the subject; and/or, (34) increasing the level of superoxide dismutase and glutathione peroxidase in the liver of a subject, reducing the level of malondialdehyde in the liver of the subject, and reducing oxidative stress in the subject during pregnancy, thereby (35) improving the pathological damaging effect on a tissue of the intestinal tract induced by abnormal diet or disease in the subject.

3. The use according to claim 1, wherein the use comprises use of an odd-carbon fatty acid in the preparation of a formulation or medicament for one or more of the following purposes:
(3) improving or enhancing the mechanical barrier of the intestinal tract of a subject, (9) maintaining the immune homeostasis in the intestinal tract (especially ileum) of the subject, (14) promoting the development of the intestinal tract of the subject and (17) maintaining the energy homeostasis of cells of the intestinal tract of the subject, and improving the energy metabolism in the intestinal tract; and/or
(21) promoting the development and metabolism of muscle tissue of a subject; and/or
(24) promoting the development and metabolism of adipose tissue in a subject; and/or
(27) reducing an allergic reaction in the body and intestinal tract of a subject.

4. The use according to claim 1, wherein the odd-carbon fatty acids are used in the form of free odd-carbon fatty acids, and/or in the form of a fatty acid chain contained in a glyceride such as a monoglyceride, a diglyceride and/or a triglyceride, the glyceride preferably being an oil or fat;
preferably, based on the total amount of the odd-carbon fatty acids, the content of C15:0 fatty acid in the odd-carbon fatty acids or glyceride is 80% or more, preferably 85% or more, 90% or more or 95% or more;
preferably, the odd-carbon fatty acids or glyceride further comprise(s) C17:0 in addition to C15:0; and based on the total weight of the odd-carbon fatty acids, the content of C17:0 is ≤ 18%, ≤ 13%, ≤ 5% or ≤ 3%.

5. The use according to claim 4, wherein the odd-carbon fatty acids are used in the form of an oil or fat containing the odd-carbon fatty acids;
preferably, in the fatty acid composition of the oil or fat, the content of the odd-carbon fatty acids is ≥ 20%, such as 20% to 70% or 20% to 60%;
preferably, in the fatty acid composition of the oil or fat: the content of C11:0 fatty acid is not higher than 5%, preferably not higher than 1%, and more preferably not higher than 0.5%; the content of C13:0 fatty acid is 1% to 5%, preferably 2% to 4%; C15:0 fatty acid is 15% to 70%, preferably 18% to 60%; the content of C17:0 fatty acid is 0.1% to 9%; the content of C19:0 fatty acid is not higher than 5%, preferably not higher than 1%, and more preferably not higher than 0.5%; the content of C21:0 fatty acid is not higher than 5%, preferably not higher than 1%, and more preferably not higher than 0.5%; and the content of C23:0 fatty acid is not higher than 5%, preferably not higher than 1%, and more preferably not higher than 0.5%.

6. The use according to claim 5, wherein the fatty acid composition of the oil or fat further comprises any one or more of C12:0, C14:0, C14:1, C16:0, C16:1, C18:0, C18:1, C18:2, C20:0, C18:3, C18:3T, C20:1, C20:2, C22:0, C20:3N6, C20:4N6, C20:5N3, C22:1, C20:3N3, C23:0, C22:2, C22:5, C22:6, C24:0 and C24:1; preferably, in the fatty acid composition of the oil or fat, the content of C16:0 is 2% to 15%, such as 4% to 13%; the content of C18:0 is ≤ 5%, such as 0% to 5% or 0% to 3%; the content of C18:1 is 5% to 35%, such as 7% to 33%; the content of C18:2 is ≤ 35%, such as 0% to 35%; the content of C22:5 is ≤ 5%, such as 0% to 5% or 1% to 3.5%; the content of C22:6 is 5% to 25%, such as 8% to 22%; and the content of the remaining fatty acids is not more than 1% or less than 0.5%.

7. The use according to claim 5, wherein:
in the fatty acid composition of the oil or fat, the content of C13:0 is 1% to 5%, preferably 2% to 4%, the content of C15:0 is 40% to 60%, preferably 45% to 53%, the content of C16:0 is 1% to 7%, preferably 3% to 5.5%, the content of C17:0 is 4% to 9%, preferably 5% to 9%, the content of C18:1 is 5% to 10%, preferably 6% to 8%, the content of C22:5 is 1% to 5%, preferably 2% to 4%, and the content of C22:6 is 15% to 25%, preferably 18% to 22%; and the content of the remaining fatty acids is less than 1% or less than 0.5%; or
in the fatty acid composition of the oil or fat, the content of C15:0 is 40% to 65%, preferably 50% to 60%, the content of C16:0 is 1% to 7%, preferably 3% to 5.5%, the content of C17:0 is 0.1% to 3%, preferably 0.1% to 1%, the content of C18:0 is 1% to 5%, preferably 1% to 3%, the content of C18:1 is 25% to 35%, preferably 28% to 34%, and the content of C18:2 is 1% to 5%, preferably 2% to 4%; and the content of the remaining fatty acids is less than 1% or less than 0.5%; or
in the fatty acid composition of the oil or fat, the content of C15:0 is 12% to 25%, preferably 16% to 22%, the content of C16:0 is 5% to 15%, preferably 10% to 15%, the content of C17:0 is 1% to 7%, preferably 2% to 5%, the content of C18:0 is 0.5% to 3%, preferably 1% to 2%, the content of C18:1 is 12% to 22%, preferably 15% to 20%, the content of C18:2 is 25% to 35%, preferably 30% to 35%, and the content of C22:6 is 5% to 15%, preferably 7% to 12%; and the content of the remaining fatty acids is less than 1% or less than 0.5%.

8. The use according to any one of claims 5-7, wherein the oil or fat is derived from an edible oil, a transesterification product thereof, or any combination of the foregoing;
preferably, the edible oil is selected from a vegetable oil, a microbial oil, an animal oil or any combination thereof;
preferably, the vegetable oil includes one or more of rice oil, sunflower seed oil (such as high-oleic sunflower seed oil), rape oil, palm oil, palm kernel oil, peanut oil, rapeseed oil, soybean oil, cottonseed oil, safflower seed oil, perilla seed oil, tea seed oil, olive oil, cacao bean oil, Triadica sebifera seed oil, sweet almond oil, almond oil, Vernicia fordii seed oil, rubber seed oil, corn oil, wheat germ oil, sesame seed oil, castor seed oil, evening primrose seed oil, hazelnut oil, pumpkin seed oil, walnut oil, grape seed oil, borage seed oil, sea buckthorn seed oil, tomato seed oil, macadimia nut oil, coconut oil and cacao butter, or a fractionation product thereof, a self-transesterification product thereof or a transesterification product of two or more kinds of oil or fat;
preferably, the animal oil is selected from one or more of lard, chicken fat, sheep fat, fish oil and beef fat;
preferably, the microbial oil includes an oil or fat derived from a microorganism selected from the group consisting of: algae, such as strains of the order Thraustochytriales, the order Thraustochytriales more specifically including strains of the genus Thraustochytrium and the genus Schizochytrium; oleaginous bacteria, such as the genus Rhodococcus, e.g., Rhodococcus opacus; oleaginous yeasts, such as the genus Yarrowia, e.g., Yarrowia lipolytica; mutant strains derived from any of the above-mentioned strains and mixed strains thereof, the microorganism preferably being a Schizochytrium sp.; and
preferably, the weight ratio of a triglyceride containing odd-carbon fatty acids for transesterification to the vegetable oil is 45-70:30-55, 50-65:35-50, or 55-62:38-45.

9. The use according to claim 8, wherein:
the oil or fat is a chemical transesterification product of the triglyceride containing odd-carbon fatty acids with a DHA algae oil and a high-oleic sunflower seed oil; preferably, in a mixture of the triglyceride containing odd-carbon fatty acids with the DHA algae oil and the high-oleic sunflower seed oil, the weight ratio of triglyceride pentadecanoate to the DHA algae oil to the high-oleic sunflower seed oil is 40-50:30-45:1-10, such as 40-50:30-40:5-10 or 40-50:38-45:1-5; preferably, in the fatty acid composition of the oil or fat, the content of C15:0 is 12% to 25%, preferably 16% to 22%, the content of C16:0 is 5% to 15%, preferably 10% to 15%, the content of C17:0 is 1% to 7%, preferably 2% to 5%, the content of C18:0 is 0.5% to 3%, preferably 1% to 2%, the content of C18:1 is 12% to 22%, preferably 15% to 20%, the content of C18:2 is 25% to 35%, preferably 30% to 35%, and the content of C22:6 is 5% to 15%, preferably 7% to 12%; and the content of the remaining fatty acids is less than 1% or less than 0.5%; or
the oil or fat is a chemical transesterification product of the triglyceride containing odd-carbon fatty acids with palm stearin and high-oleic sunflower seed oil; preferably, in a mixture of the triglyceride containing odd-carbon fatty acids with the palm stearin and the high-oleic sunflower seed oil, the weight ratio of triglyceride pentadecanoate to the palm stearin to the high-oleic sunflower seed oil is 55-62:1-5:35-40; in the fatty acid composition of the oil or fat, the content of C15:0 is 40% to 65%, preferably 50% to 60%, the content of C16:0 is 1% to 7%, preferably 3% to 5.5%, the content of C17:0 is 0.1% to 3%, preferably 0.1% to 1%, the content of C18:0 is 1% to 5%, preferably 1% to 3%, the content of C18:1 is 25% to 35%, preferably 28% to 34%, and the content of C18:2 is 1% to 5%, preferably 2% to 4%; and the content of the remaining fatty acids is less than 1% or less than 0.5%.

10. An oil or fat according to any one of claims 4-9.

11. A formulation comprising the odd-carbon fatty acids, or oil or fat, as mentioned in any one of claims 1-9; preferably, the formulation is a dietary supplement.
